# EUROPEAN PATENT SPECIFICATION

(11) **EP 4 171 512 B1**
(45) Date of publication and mention of the grant of the patent: **28.01.2026**
(21) Application number: 21746185.4
(22) Date of filing: 22.06.2021
(51) Int. Cl.: A61K 9/08, A61K 47/10, A61K 47/14, A61K 47/26, A61K 31/4045

(54) **ORAL LIQUID FORMULATIONS OF RUXOLITINIB**
ORALE FLÜSSIGE FORMULIERUNGEN VON RUXOLITINIB
FORMULATIONS LIQUIDES ORALES DE RUXOLITINIB

(30) Priority: 24.06.2020 IN 202011026726
(43) Date of publication of application: 03.05.2023
(73) Proprietor: Novartis AG, 4056 Basel (CH)
(72) Inventor: BORDE, Parag, Hyderabad, 500101 (IN); CHERKUPALLY, Laxman, Hyderabad, 500101 (IN); KARPINSKI, Paul, East Hanover, New Jersey 07936 (US); RATHOD, Akash, Hyderabad, 500101 (IN)
(74) Representative: Ridout, Joseph
(86) International application number: PCT/IN2021/050604
(87) International publication number: WO 2021/260727

(56) References cited:
- WO-A1-2019/021229
- WO-A2-2015/184087
- WO-A2-2019/224803

## Description

### Field of the Invention

Compositions and technologies relating to an oral liquid formulation of ruxolitinib.

### Background of the Invention

Ruxolitinib, (R)-3-(4-(7H-pyrrolo[2,3-d]pyrimidin-4-yl)-1H-pyrazol-1-yl)-3-cyclopentylpropanenitrile, is a marketed product that is formulated as an oral immediate release tablets administered twice-a-day (BID) in 5-mg, 10-mg, 15-mg, 20-mg, and 25-mg dose strengths for treatment of Janus kinase (JAK) associated diseases, such as myelofibrosis (MF), polycythemia vera (PV) and graft-versus-host disease (GvHD) for adult patients. The formulation of ruxolitinib addressed herein supplements the existing products by providing a dosage form that is in liquid form.

GvHD is prevalent in pediatric patients, 28 days to 18 years old, thus an age-appropriate dosage form is desired. In addition to the pediatric population, there are also adult patients are that have difficulty swallowing solid dosage form of ruxolitinib. Accordingly, there is a need for new and improved formulations of various doses of ruxolitinib to improve safety and to increase patient compliance.

WO2015/184087 is directed to the combination of JAK2 inhibitors with thalidomide derivatives and glucocorticoids.

WO2019/021229 is directed to liquid dosage forms comprising the ABL kinase inhibitor imatinib.

WO2019/224803 is directed to therapeutic methods and compositions for treating myeloproliferative neoplasms, including inter alia to combination therapy involving specific MDM2 inhibitors.

### Summary of the Invention

An aspect of the present invention provides an oral liquid formulation comprising:
(i) ruxolitinib, also known as (R)-3-(4-(7H-pyrrolo[2,3-d]pyrimidin-4-yl)-1H-pyrazol-1-yl)-3-cyclopentylpropanenitrile, of formula I: 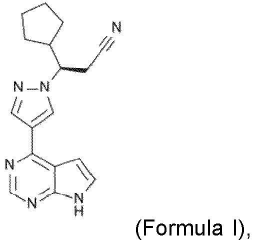 or a pharmaceutically acceptable salt thereof; (ii) water; (iii) a pH adjusting agent; and(iv) a preservative; wherein the ruxolitinib concentration in the oral formulation is from about 0.1 mg/mL to about 10 mg/mL on a free base basis, and wherein the pH of the oral formulation 2.7 ± 0.5.

In one embodiment, the preservative in the oral formulation is a mixture of methyl paraben and propyl paraben.

In one embodiment, the oral formulation further comprises a co-solvent.

In one embodiment, the oral formulation further comprises a sweetner.

In one embodiment, the oral formulation further comprises a flavouring agent.

In one embodiment, the co-solvent is propylene glycol.

In one embodiment, the sweetner is sucralose.

In one embodiment, the flavour is strawberry flavour.

In one embodiment, the pH-adjusting agent is citric acid.

In one emobidment, the ruxolitinib (on a free base basis) concentration is about 1 mg/mL to 5 mg/mL.

Another aspect of the present invention provides the above mentioned formulation of the invention for use in a method of treating a Janus kinase (JAK) associated disease comprising the step of administering the oral formulation of the presention disclosure, comprising about 1 mg to about 65 mg of ruxolitinib, on a free base basis, or pharmaceutically acceptable salt thereof.

### Brief Description of the Drawing

Figure 1 summarizes the study design of the pediatric acute GvHD trial.
Figure 2 summarizes the study design of the pediatric chronic GvHD trial.

### Detailed Description of the Invention

The current commercial formulation of Jakafi/Jakavi^{™} (ruxolitinib) is an oral tablet, which is administered twice-a-day (BID).

Ruxolitinib phosphate has been designated a Class 1 compound in the Biopharmaceutical Classification System (BCS). Ruxolitinib phosphate is a water-soluble drug with pH dependent solubility exhibiting increased solubility at lower pH. The drug substance in liquid shows degradation mainly due to oxidation. Temperature and oxygen triggers an increase in oxidative degradation. For an oral solution formulation, the pH needs to be maintained between 2.4 to 4.0 with an acidifying agent such as, citric acid to keep the drug in solution. Nitrogen purging is conducted during the manufacturing process at the drug dispersion step to provide an inert environment with minimal oxygen. Maintaining the pH of the solution at 2.4-4.0 with the right preservative system, such as methyl paraben plus propyl paraben, taste masking agent, such as sucralose, a flavouring agent such as strawberry flavour, and a simple method of mixing was found to be effective resulting in a stable palatable oral solution formulation.

The formulation is stable based on chemical/physical stability with a current shelf life of 12 months at room temperature (shelf life is expected to be extended as additional stability data becomes available) and microbial stability with optimum levels of preservatives and safety levels of excipients.

This formulation can be stored at room temperature in contrast to cool temperature where most of the liquid dosage forms are stored. It is a sugar free, multiple-use formulation that can be given to pediatric patients for chronic use without much concern on dental carries. It can also be given to adult patients who has difficulty swallowing solid dosage forms.

The dose of ruxolitinib administered to a patient depends on numerous factors such as weight of patient, body surface area of the patient, the severity of symptoms and the nature of any other drugs being administered. Therefore the therapeutic dose was adjusted accordingly to achieve comparable pharmacokinetic profile and similar therapeutic effect.

The formulation is an immediate release formulation. It is usually adminisered twice-a-day (BID).

As used herein the term "pharmaceutical composition" means a mixture (e.g., a solid dispersion) and/or solution (e.g., a liquid solution) containing a therapeutic compound to be administered to a mammal, e.g., a human in order to prevent, treat or control a particular disease or condition affecting the mammal. The term "pharmaceutical composition" as used herein, for example, also encompasses an intimate physical mixture formed at high temperature and pressure.

As used herein the term "pharmaceutically acceptable" refers to those compounds, materials, compositions and/or dosage forms, which are, within the scope of sound medical judgment, suitable for contact with the tissues of mammals, especially humans, without excessive toxicity, irritation, allergic response and other problem complications commensurate with a reasonable benefit/risk ratio.

As used herein the term "therapeutic compound" means any compound, substance, drug, medicament, or active ingredient having a therapeutic or pharmacological effect, and which is suitable for administration to a mammal, e.g., a human, in a composition that is particularly suitable for oral administration. Particularly useful as a therapeutic compound in the present invention is ruxolitinib and pharmaceutically acceptable salts thereof.

As used herein the term "ruxolitinib" refers to the free base of ruxolitinib or a pharmaceutically acceptable salt thereof (e.g., ruxolitinib phosphate).

In an exemplary embodiment, the oral formulation contains ruxolitinib phosphate salt.

The oral formulation of the invention comprises one or more preservative(s). The preservatives can be antimicrobial preservatives, alone or in combination. The preservative(s) can be selected from the group consisting of potassium sorbate, sodium benzoate, methyl paraben and propyl paraben.

In one particular embodiment, the preservative is a mixture of methyl paraben and propyl paraben.

The ratio of methyl paraben and propyl paraben in the preservative mixutre can vary from 3:1 to 1:9 (weight ratio).

The conceration range of preservative in the formulation is from abont 0.1 mg/mL to 5 mg/mL. As an example, the preservative methyl paraben concentration is about 1.2-1.3 mg/mL and propyl paraben concentration is 0.3-0.5 mg/mL.

In another embodiment, the oral formulation comprises one or more co-solvent(s). The co-solvent(s) can be selected from the group consisting of ethanol, glycerol, propylene glycol and, polyethylene glycol.,

In one particular embodiment, the co-solvent is propylene glycol.

The conceration range of co-solvent(s) in the formulation is from abont 50 mg/mL to 300 mg/mL. As an example, the co-solvent concentration is about 100-200 mg/mL, more specifically, 150 mg/mL.

In another embodiment, the oral formulation comprises one or more sweetner(s) to mask unpleasant organoleptic properties of drug or excipients.The sweetner(s) can be selected from the group consisting of sucrose, sucralose, aspartame, Acesulfame potasium and sodium saccharin.

In one particular embodiment, the sweetner is a sucralose.

The conceration range of sweetner in the formulation is from abont 0.5mg/mL to 6mg/mL. As an example, the sweetner concentration is about 1-3 mg/mL, more specifically, 2mg/mL.

In another embodiment, the oral formulation comprises one or more flavouring agents to mask unpleasant organoleptic properties of drug or excipients.The flavouring agents can be selected from the group consisting of flavours of banana, chocolate, orange, cherry, strawberry, blueberry, tutti fruity etc.

In one particular embodiment, the flavoring agent is a strawberry flavour.

The conceration range of flavouring agent in the formulation is from about 0.5 mg/mL to 5 mg/mL. As an example, the strawberry flavour concentration is about 1-4 mg/mL, more specifically, 2.5 mg/mL.

The oral formulation of the invention comprises one or more pH adjusting agent(s). The pH adjusting agent(s) can be selected from the group consisting of citric acid, fumaric acid, lactic acid, phosporic acid, tartaric acid and succinic acid.

In one particular embodiment, the pH-adjusting agent is citric acid.

In an embodiment, the concentration range of pH-adjusting agent in the formulation is from abont 2 mg/mL to 8 mg/mL. As an example, the pH-adjusting agent range is about 4-5 mg/mL, specifically about 4.27 mg/mL.

The pH level of the oral formulation of the invention is 2.7±0.5.

The ruxolitinib (on the free base basis) concentration in oral formulation of the invention is from about 0.1 mg/mL to about 10 mg/mL, preferrably from about 1mg/mL to 6mg/mL, most preferably at about 1mg/mL to 5mg/mL.

### Methods

Another aspect of the present invention pertains to the formulation of the invention for use in methods of treating a JAK-associated disease or disorder in an individual (e.g., patient) by administering to the individual in need of such treatment the oral liquid formulation of the invention. A JAK-associated disease can include any disease, disorder or condition that is directly or indirectly linked to expression or activity of the JAK, including overexpression and/or abnormal activity levels. A JAK-associated disease can also include any disease, disorder or condition that can be prevented, ameliorated, or cured by modulating JAK activity.

In further embodiments, the JAK-associated disease is cancer including those characterized by solid tumors (e.g., prostate cancer, renal cancer, hepatic cancer, pancreatic cancer, gastric cancer, breast cancer, lung cancer, cancers of the head and neck, thyroid cancer, glioblastoma, Kaposi's sarcoma, Castleman's disease, uterine leiomyosarcoma, melanoma etc.), hematological cancers (e.g., lymphoma, leukemia such as acute lymphoblastic leukemia (ALL), acute myelogenous leukemia (AML) or multiple myeloma), and skin cancer such as cutaneous T-cell lymphoma (CTCL) and cutaneous B-cell lymphoma. Example CTCLs include Sezary syndrome and mycosis fungoides.

JAK-associated diseases can further include myeloproliferative disorders (MPDs) such as polycythemia vera (PV), essential thrombocythemia (ET), primary myelofibrosis (PMF), chronic myelogenous leukemia (CML), chronic myelomonocytic leukemia (CMML), hypereosinophilic syndrome (HES), systemic mast cell disease (SMCD), and the like. In some embodiments, the myeloproliferative disorder is myelofibrosis (e.g., primary myelofibrosis (PMF) or post polycythemia vera/essential thrombocythemia myelofibrosis (Post-PV/ET MF)). In some embodiments, the myeloproliferative disorder is post-essential thrombocythemia myelofibrosis (Post-ET MF). In some embodiments, the myeloproliferative disorder is post polycythemia vera myelofibrosis (Post-PV MF).

JAK-associated diseases can further include graft vs. host disease (GvHD) such as acute graft vs. host disease (aGvHD) and chronic graft vs. host disease (cGvHD)

### EXAMPLES

The following examples illustrate aspects of the invention and are not a limitation on the present invention. Formulations for preparing the oral solution are set out below.

### Example 1: Stable Pediatric Oral Solution Formulation at 5 mg/mL concentration

**Table 1. Composition of the formulation**

| **Component** | **Functionality** | **Amount per unit (mg/mL)** |
|---|---|---|
| Ruxolitinib phosphate* | Active ingredient | 6.600 |
| Propylene glycol | Co-solvent | 150.000 |
| Methyl paraben | Preservative | 1.200 |
| Propyl paraben | Preservative | 0.400 |
| Sucralose | Sweetener | 2.000 |
| Citric acid | pH-adjusting agent | 4.270 |
| Strawberry flavor | Flavor | 2.500 |
| Purified water | Vehicle | 848.030 |
| Total | | q.s to 1mL |

| | | |
|---|---|---|
| * Salt factor: 1.320 | | |

This stable pediatric oral solution (Table 1) has been developed and has a current shelf life of 12 months at room temperature with the potential for extension as additional stability data becomes available. Ruxolitinib phosphate tend to form co-crystals with sodium benzoate, a preservative. The combination of methyl paraben and propyl paraben, as a preservative system, has been found to be acceptable in the current oral solution formulation. The manufacturing process is a simple mixing process with several mixing substeps to dissolve components prior to mixing together and does not require any special process or technology. The taste of the drug substance is mostly masked with use of sucralose, a sweetener and flavours like strawberry flavour. The excipients and their levels in the formulation are acceptable for children age >1 month to 18 years from the safety perspective.

### Example 2: Key characterization data demonstrating technical superiority and / or unexpected effects

Development of stable and palatable oral solution formulation for ruxolitinib is challenging due to pH dependent solubility (see Table 2) of the drug substance with mainly oxidation degradation products at higher temperatures/moisture content and/or in presence of oxygen. As illustrated in Table 2 below, ruxolitinib phosphate shows pH-dependent solubility in aqueous medium.

**Table 1 Solubility rofile of ruxolitinib phosphate**

| **Buffer solution** | **Solubility (mg/ml) at 37°C** |
|---|---|
| pH 1.0 | ≥ 0.54 |
| pH 3.3 | ≥ 0.52 |
| pH 4.3 | 0.35 |
| pH 5.3 | 0.29 |
| pH 7.5 | 0.15 |
| pH 8.0 | 0.17 |

In order to keep the drug in solution (1 and 5 mg/mL), citric acid, a palatible pH-modifying agent was chosen. Citric acid at 0.43% was selected as pH modifying agent to maintain the drug in solution at pH 2.7±0.3.

Forced degradation of initial formulations was performed to check the degradation profile of the solution. Forced degradation was carried out by using acid (0.1N HCl), base (0.1N NaOH), high temperature like 60 °C and oxidation (3% H₂O₂) as main parameters. There were increased levels of two major degradants 521-11 and 536-11 (Table 3).

The structure of 521-11 is

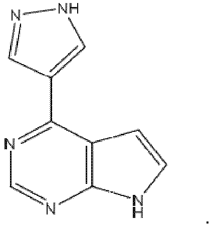

The structure of 536-11 is

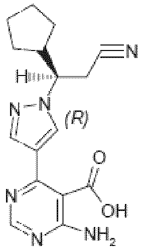

**Table 0 Forced degradation study of ruxolitinib phosphate**

| | % Degradation products | | | |
|---|---|---|---|---|
| Condition | 1 mg/ml solution formulation | | 5 mg/ml solution formulation | |
| | 521-11 | 536-11 | 521-11 | 536-11 |
| | | | | |
| Initial | 0.1231 | NR | 2.564 | NR |
| Acid/RT | 0.0989 | 0.2075 | 0.094 | NR |
| Acid/Oven | 1.4302 | 0.3028 | 0.1345 | 0.2252 |
| Oven | 0.1025 | NR | 0.1009 | NR |
| Base/RT | 0.5095 | NR | 0.1569 | NR |
| Base/oven | 1.1733 | NR | 0.669 | NR |
| Oven | 0.0789 | NR | 0.1009 | NR |
| Peroxide | 0.0604 | NR | 3.0027 | 1.2355 |

The stability data of the 5mg/mL oral solution formulation in two fill volumes (60mL fill in 125mL bottle and 140mL fill in 300mL bottle) has been generated and found to be acceptable (Table 4).

**Table 2 Stability data of formulations**

| Batch/ Fill volume | Condition | Assay of drug % [90-110] | Assay of MP % [90-110] | Assay of PP % [90-110] | Unspecified at RRT 0.3* % [≤ 0.5] | Unspecified at RRT 0.5** % [≤ 0.5] | Enantiomer % [≤ 0.7] | Total impurities % [≤ 2.0] |
|---|---|---|---|---|---|---|---|---|
| | Initial | 102.4 | 101.4 | 100.4 | < RT | < RT | 0.2 | 0.2 |
| | 5°C -3M | 101.7 | 100.3 | 100.4 | < RT | < RT | 0.2 | 0.2 |
| H0004 (60ml) | 25°C/60% RH-3M | 101.5 | 99.2 | 99.4 | < RT | < RT | 0.2 | 0.2 |
| | 40°C/75% RH-3M | 101.1 | 97.3 | 97.9 | 0.3 | 0.1 | 0.2 | 0.7 |
| H0004 (140ml) | Initial | 103.5 | 101.4 | 100.5 | < RT | < RT | 0.2 | 0.2 |
| | 5°C-3M | 101.4 | 100.0 | 99.9 | < RT | < RT | 0.2 | 0.2 |
| 25°C/60% RH -3M | 101.2 | 99.2 | 99.4 | < RT | < RT | 0.2 | 0.2 | |
| 40°C/75% RH-3M | 99.8 | 97.8 | 98.7 | 0.3 | 0.2 | 0.2 | 0.7 | |

| | | | | | | | | |
|---|---|---|---|---|---|---|---|---|
| * This impurity has been confirmed as 4-hydroxyl benzoic acid, oxidation product of parabens. ** This impurity has been confirmed as 536-11 as an oxidative degradation product of ruxolitinib phosphate | | | | | | | | |

The oral solution (5mg/mL) is found to be stable at room temperature with acceptable taste safe levels of excipients with simple manufacturing process.

### Example 3: A Phase I/II open-label, single-arm, multi-center study of ruxolitinib added to corticosteroids in pediatric patients with grade II-IV acute graft vs. host disease after allogeneic hematopoietic stem cell transplantation

### Purpose:

Acute graft vs. host disease (aGvHD) pathophysiology begins with activation of host APC which in turn present host antigens to donor immune cells, leading to donor T-cell proliferation and inflammatory cytokine production. These inflammatory cytokines then recruit and induce proliferation of additional immune effector cells, thereby perpetuating an adverse cycle of allo-reactive tissue injury and inflammation (Paczesny et al 2010). Ruxolitinib has been shown to lower pro-inflammatory cytokines in MF patients. In addition, pre-clinical data support the mechanism of action of ruxolitinib in GvHD to: i.) impair APC function, ii.) inhibit donor T cell proliferation, iii.) suppress adverse cytokine production, and iv.) improve survival and disease manifestations in GvHD mouse models (Parampalli Yajnanarayana et al 2015, Heine et al 2013, Spoerl et al 2014). Further, recently published data have shown evidence of clinical efficacy with ruxolitinib treatment when added to immunosuppressive therapy in patients with SR-aGvHD (Zeiser et al 2015; Spoerl et al 2014). Clinical studies using ruxolitinib alone or in comparison to best available therapy are currently underway in the SR-aGvHD setting for adult patients and a proportion of adolescents ≥ 12 years of age.

While children are at less risk of developing aGvHD than adults, that risk is still significant especially when using alternative donor sources (Jacobsohn and Vogelsang 2007). Similar to the adults, there are limited treatment options for Grade II-IV aGvHD including systemic corticosteroids as initial standard of care for pediatric patients. With only 30-50% of the children responding to corticorteroids, there is a high unmet medical need for optimal initial and second-line therapies in the pediatric population. Recent data with ruxolitinib in SR-aGvHD pediatric patients have shown encouraging overall response rates compared to corticosteroids +/- CNI alone. Given this data (presented above) in the current setting of a lack of effective first- or second-line treatments for pediatric aGvHD, the study intends to assess safety, activity and pharmacokinetics of ruxolitinib treatment with corticosteroids in treatment-naïve and SR- aGvHD patients aged ≥28 days up to <18 years of age. It is expected that ruxolitinib will provide higher rates of disease response compared to steroids +/- CNI alone as upfront treatment of grade II-IV aGvHD. It is further expected that this response will be durable during steroid taper, representing a meaningful clinical benefit for patients.

Furthermore, treatment-naive patients could potentially benefit from the steroid-sparing effect due to the risks associated with long-term exposure and toxic effects of steroids observed in children. The expected mechanism of action as well as the safety profile of ruxolitinib in both treatment-naïve and SR-aGvHD are similar, given that the treatment-naive patients have not been subjected to extensive pre-treatment for the underlying disease as compared to adults.

Expected meaningful clinical benefits in patients treated with ruxolitinib include its steroid sparing effect, reducing the proportion of patients experiencing flares during steroid tapering, reducing proportion of patients with infections and severity of infections, reducing hospitalization duration and requirement for re-admission, maintenance of graft vs. malignancy effect, and possible reduction of the proportion of patients developing cGvHD.

### Objectives and endpoints

**Table 5 Objectives and related endpoints**

| **Objective(s)** | | **Endpoint(s)** |
|---|---|---|
| **Primary objective(s)** | | **Endpoint(s) for primary objective(s)** |
| • Phase I | | • Measurement of PK parameters in aGvHD and SR-aGvHD patients: AUC, Cmax, T1/2, Ctrough using extensive PK sampling in Groups 1-3 and sparse sampling in Group 4 |
| | To assess pharmacokinetic (PK) parameters of ruxolitinib for patients with aGvHD and SR-aGvHD and define an age appropriate RP2D for each of the groups 2-4 | |
| | | • Age-based determination of RP2D for each of the groups 2-4, based on observed PK parameters. |
| | • Group 2: age ≥6y to <12y | |
| | • Group 3: age ≥2y to <6y | |
| | • Group 4: age ≥28 days to < 2y | |
| • Phase II | | • Overall response rate (ORR) at Day 28, defined as the proportion of patients demonstrating a complete response (CR) or partial response (PR) without requirement for additional systemic therapies for an earlier progression, mixed response or non-response. Scoring of response will be relative to the organ stage at the start of the study treatment. |
| | To measure the activity of ruxolitinib in patients with aGvHD or SR-aGvHD assessed by Overall Response Rate (ORR) at Day 28. | |

| **Secondary objective(s)** | | **Endpoint(s) for secondary objective(s)** |
|---|---|---|
| • **Key Secondary** | | • Proportion of all patients who achieve a CR or PR at Day 28 and maintain a CR or PR at Day 56 |
| | To assess the rate of durable ORR at Day 56 | |
| • To estimate ORR at Day 14 | | • Proportion of patients who achieved OR (CR+PR) at Day 14 |
| • To assess pharmacokinetic/pharmacodynamic relationships | | • PK parameters (such as AUC, Cmax, Ctrough) versus safety, efficacy, and PD biomarkers, as appropriate |
| • To assess Duration of response | | • Duration of response (DOR) is assessed for responders only and is defined as the time from first response until aGvHD progression or the date of additional systemic therapies for aGvHD. Onset of chronic GvHD, or death without prior observation of aGvHD progression are considered as competing risks |
| • To assess the cumulative steroid dose until Day 56 | | • Weekly cumulative steroid dose for each patient up to Day 56 |
| • To evaluate the safety and tolerability of ruxolitinib | | • Safety and tolerability including myelosuppression, infections, and bleeding will be assessed by monitoring the frequency, duration and severity of Adverse Events including occurrence of any second primary malignancies, infections, by performing physical exams, and evaluating changes in vital signs from baseline, routine serum chemistry, hematology results and coagulation profile |
| • To assess Overall Survival (OS) | | • Overall survival, defined as the time from the start of treatment to the date of death due to any cause |
| • To assess Event-Free Survival (EFS) | | • Event-free survival, defined as the time from start of treatment to the date of hematologic disease relapse/progression, graft failure, or death due to any cause |
| • To assess Failure-Free Survival (FFS) | | • Failure-free survival, defined as the time from the start of treatment to date of hematologic disease relapse/progression, non-relapse mortality, or addition of new systemic aGvHD treatment |
| • To assess Non Relapse Mortality (NRM) | | • Non-relapse mortality (NRM), defined as the time from start of treatment to date of death not preceded by hematologic disease relapse/progression |
| • To assess incidence of Malignancy Relapse/Progression (MR) | | • Malignancy Relapse/Progression (MR), defined as the time from start of treatment to hematologic malignancy relapse/progression. Calculated for patients with underlying hematologic malignant disease |
| • To measure the incidence of cGvHD | | • cGvHD, defined as the diagnosis of any cGvHD including mild, moderate, severe |
| • To estimate the rate of Best Overall Response (BOR) | | • Proportion of patients who achieved OR (CR+PR) at any time point up to and including Day 28 and before the start of additional systemic therapy for aGvHD |
| • To assess graft failure | | • Monitoring of donor cell chimerism, defined as initial whole blood or marrow donor chimerism >5% declining to <5% on subsequent measurements compared to baseline |
| • To describe the acceptability and palatability assessments of the ruxolitinib formulation | | • Questionnaire on acceptability and palatability for dose forms used after first dose, 1 month and 6 months |

### Study design

This open-label, single-arm, Phase I/II multi-center study will investigate the PK, activity and safety of ruxolitinib added to the patient's immunosuppressive regimen in infants, children, and adolescents ages ≥28 days to <18 years old with either grade II-IV aGvHD or grade II-IV SR-aGvHD. This trial will utilize age groups: Group 1 includes patients ≥12y to <18y, Group 2 includes patients ≥6y to <12y, Group 3 includes patients ≥2y to <6y, and Group 4 includes patients ≥28days to <2y. Patients will remain in the age group throughout the study based on the age at the time of start of treatment. Enrollment initiation into the youngest age group, Group 4 (Phase I/II) will be subject to the review of available PK, safety, and activity data in consultation with the data monitoring committee (DMC), Pediatric Committee (PDCO), and a final decision by the Sponsor.

All patients will be enrolled and treated for 24 weeks (approximately 6 months) or until early discontinuation. All patients will also be followed for additional 18 months (total duration = 2 years from enrollment). Should the occurrence of aGvHD flare require treatment re-initiation or should ruxolitinib not be discontinued by the end of 24 weeks due to extended tapering, patients may continue taper ruxolitinib beyond 24 weeks up to a maximum of 48 weeks. As patients ≥12 y to < 18 y (Group 1) are already being included in trial [CRUXOLITINIBC2301], and treated with 10 mg BID, this dose is the recommended phase II dose (RP2D), and will be used to treat all patients in this age group. For the Ph II, all other age groups will be treated with the RP2D determined during the Ph I. Therefore, all ≥12 to <18 year old patients will automatically be enrolled in Phase II. It is planned that the first 5 patients treated in Group 1 will undergo extensive PK sampling to inform the RP2D determination of the younger age groups in the Ph I. Additional patients may undergo extensive sampling should one or more of the first 5 not be evaluable.

### Phase I

Patients will be enrolled into 4 groups, based on age, to allow appropriate dosing based on available data Table 2.
- Phase I (Fig. 1): Full ruxolitinib concentration-time courses, safety and activity data will be collected over 28 days for Groups 2, 3 and 4. Groups 2 and 3 will be enrolled initially, and the PK data generated from all patients (including Group 1) will be used to inform the starting dose of Group 4. Therefore, Group 4 will only open after an RP2D has been defined for Group 2, and an RP2D has been defined for Group 3.
- The PK and safety data will be used to assess the adequacy of the preliminary starting dose, which can be adapted if needed (i.e. to account for any potential difference between the expected and the observed ruxolitinib exposure).
- Should the exposure in Groups 2, 3 or 4 not be confirmed following the PK sampling in 5 evaluable patients, an additional 5 patients will be enrolled in that specific age group until the dose/exposure is confirmed (i.e. selection of the RP2D for those ages based on exposure and safety review by the DMC).

**Table 6 Phase I: Age groups and dosing rationale**

| **Group** | **Group 1** | **Group 2** | **Group 3** | **Group 4** |
|---|---|---|---|---|
| Age Range | ≥12y to < 18y | ≥6y to < 12y | ≥2y to < 6y | ≥28 days to < 2y |

| N (Ph I) | Not applicable | 5 evaluable | 5 evaluable | Undefined |
|---|---|---|---|---|
| Preliminary dose | | PBPK-derived equivalent predicted to yield similar exposure to 10 mg BID adult dose =5 mg BID | PBPK-derived equivalent predicted to yield similar exposure to 10 mg BID adult dose =4 mg/m² BID | Will be generated based on PK data from groups 1-3 |

### Inclusion criteria

Subjects eligible for inclusion in this study must meet all of the following criteria:
1. Male or female patients age ≥28 days and <18 years at the time of informed consent.
2. Patients who have undergone alloSCT from any donor source (matched unrelated donor, sibling, haplo-identical) using bone marrow, peripheral blood stem cells, or cord blood. Recipients of myeloablative or reduced intensity conditioning are eligible.
3. Patients with a clinically confirmed diagnosis of grades II-IV aGvHD within 48 hours prior to study treatment start. Patients may have either:
   Treatment-naive grades II-IV aGvHD as per Harris et al 2016.
      OR
   Steroid refractory grades II-IV aGvHD as per institutional criteria, and the patient is currently receiving systemic corticosteroids.
4. Evident myeloid engraftment with absolute neutrophil count (ANC) > 1000/µl and platelet count >20,000/µl. (Use of growth factor supplementation and transfusion support is allowed.)
5. Able to swallow study medication.
6. Written Study Informed consent and/or assent from the patient, parent, or guardian at the time of Screening, i.e. at the time of treatment-naïve aGvHD or steroid refractory aGvHD diagnosis.

### Exclusion criteria

Subjects meeting any of the following criteria are not eligible for inclusion in this study.
1. Has received the following systemic therapy for aGvHD:
   a. Treatment-naive aGvHD patients have received any prior systemic treatment of aGvHD except for a maximum 72h of prior systemic corticosteroid therapy of methylprednisolone or equivalent after the onset of acute GvHD. Patients are allowed to have received prior GvHD prophylaxis which is not counted as systemic treatment (as long as the prophylaxis was started prior to the diagnosis of aGvHD);
      OR
   b. SR-aGvHD patients have received two or more prior systemic treatment for aGvHD in addition to corticosteroids.
2. Clinical presentation resembling de novo chronic GvHD or GvHD overlap syndrome with both acute and chronic GvHD features (as defined by Jagasia et al 2015).
3. Failed prior alloSCT within the past 6 months.
4. Presence of clinically active uncontrolled infection including significant bacterial, fungal, viral or parasitic infection requiring treatment. Infections are considered controlled if appropriate therapy has been instituted and, at the time of screening, no signs of progression are present. Progression of infection is defined as hemodynamic instability attributable to sepsis, new symptoms, worsening physical signs or radiographic findings attributable to infection. Persisting fever without other signs or symptoms will not be interpreted as progressing infection.
5. Evidence of uncontrolled Hepatitis B Virus (HBV) or Hepatitis C Virus (HCV) based on assessment of treating physician.
6. Evidence of clinically active tuberculosis (clinical diagnosis per local practice; skin testing is not required as not informative due to anergy).
7. Known human immunodeficiency virus infection (HIV).
8. Presence of relapsed primary malignancy, or who have been treated for relapse after the alloSCT was performed, or who require withdrawal of immune suppression as pre-emergent treatment of early malignancy relapse.
9. Acute GvHD occurring after non-scheduled DLI administered for pre-emptive treatment of malignancy recurrence. Note: Patients who have received a scheduled DLI as part of their transplant procedure and not for management of malignancy relapse are eligible.
10. Significant respiratory disease including patients who are on mechanical ventilation or who have resting O2 saturation <90% by pulse-oximetry on room-air.
11. Presence of severely impaired renal function (confirmed within 72 hrs prior to study treatment start) defined by:
   - Glomerular Filtration Rate (GFR) < 30 mL/min/1.73 m2 using estimated creatinine clearance calculated by updated bedside Schwartz equation or Cockroft Gault equation
      OR
   - Renal dialysis requirement
12. Clinically significant or uncontrolled cardiac disease including any of the following:
   - Acute myocardial infarction within 6 months from Day 1 of study treatment administration
   - Uncontrolled hypertension
   - New York Heart Association Class III or IV congestive heart failure
   - Unstable angina within last 6 months from screening
   - Clinically significant (symptomatic) cardiac arrhythmias (e.g., sustained ventricular tachycardia, and clinically significant second or third degree AV block without a pacemaker, circulatory collapse requiring vasopressor or inotropic support, or arrhythmia requiring therapy).
13. Cholestatic disorders, or unresolved sinusoidal obstructive syndrome/veno-occlusive disease of the liver (defined as persistent bilirubin abnormalities not attributable to aGvHD and ongoing organ dysfunction).
14. History of bone disorders such as osteogenesis imperfecta, rickets, renal osteodystrophy, osteomyelitis, osteopenia, fibrous dyslpasia, osteomalacia etc. prior to the underlying diagnosis which resulted in the alloSCT.
15. History of endocrine or kidney related growth retardation prior to the underlying diagnosis which resulted in the alloSCT.
16. Any corticosteroid therapy for indications other than aGvHD at doses > 1 mg/kg/day methylprednisolone (or equivalent prednisone dose 1.25 mg/kg/day) within 7 days of Screening. Routine corticosteroids administered during conditioning or cell infusion is allowed.
17. Current therapy with medications that interfere with coagulation or platelet function including but not limited to aspirin and related drugs, heparin, and warfarin (to minimize risk of bleeding). Note: Heparin or Low Molecular Weight Heparin (LMWH) is allowed if used at sub-therapeutic dose for e.g., prophylaxis of sinusoidal obstructive syndrome/veno-occlusive disease of the liver.
18. History of progressive multifocal leuko-encephalopathy (PML).
19. Patients who received JAK inhibitor therapy for any indication after initiation of current alloSCT conditioning.
20. Investigational treatment within 30 days prior to treatment initiation or within 5 half-lives of the investigational product, whichever is greater.
21. Any condition that would, in the Investigator's judgment, interfere with full participation in the study, including administration of study treatment and attending required study visits; pose a significant risk to the patient; or interfere with interpretation of study data.
22. Known allergies, hypersensitivity, or intolerance to systemic immunosuppressive therapy or ruxolitinib (or any of its excipients).
23. Female patients who are pregnant or breast feeding.
24. Female patients of childbearing potential (e.g., are menstruating) who do not agree to abstinence or, if sexually active, do not agree to the use of contraception.

### Study treatment

The study treatment will be administered as a 5 mg tablet or as an oral pediatric formulation (taken in liquid form) twice a day for all patients.
- Treatment-naïve aGvHD: In addition to ruxolitinib, treatment MUST include methylprednisolone (or equivalent prednisone) +/- cyclosporine or tacrolimus at standard dosing adjusted to therapeutic trough levels
- SR-aGVHD: In addition to ruxolitinib, concomitant use of corticosteroids +/cyclosporine or tacrolimus at standard dosing adjusted to therapeutic trough levels is allowed.

In addition to study treatment, patients may receive standard alloSCT supportive care including anti-infective medications and transfusion support. Continued use of systemic corticosteroids, CNI (cyclosporine or tacrolimus), and topical corticosteroid therapy per institutional guidelines is permitted. Other systemic medications used for prophylaxis of aGvHD may be continued after Day 1 only if prior to diagnosis of aGvHD. For SR-aGvHD patients, cessation of other systemic treatment for aGvHD other than corticosteroids +/- CNI will be required prior to treatment initiation.

**Table 7 Dose and treatment schedule**

| **Study treatments** | **Age groups** | **Pharmaceutical form and Route of Administration** | **Starting dose** | **Frequency and/or Regimen** |
|---|---|---|---|---|
| Ruxolitinib | Group 1 | 5-mg tablet for oral use OR oral pediatric formulation of Example 1* | 10 mg BID (2 tablets orally BID) OR 10 mg BID oral pediatric formulation of Example 1** | Twice per day |
| Ruxolitinib | Group 2 | 5-mg tablet for oral use OR oral pediatric formulation of Example 1* | 5 mg BID (1 tablet orally BID) OR 5 mg BID oral pediatric formulation of Example 1** | Twice per day |
| Ruxolitinib | Group 3 | 5-mg tablet for oral use OR oral pediatric formulation of Example 1* | 4 mg/m² BID (either tablet orally OR oral pediatric formulation of Example 1)** | Twice per day |
| Ruxolitinib | Group 4 | 5-mg tablet for oral use OR oral pediatric formulation of Example 1* | To be defined | Twice per day |
| *Note: Tablet for oral use may be crushed as per instructions in the pharmacy manual (if calculated dose based on BSA is not 5 mg or 10 mg, then crushing is not permitted. In this case, oral pediatric formulation should be administered (taken in liquid form)). Oral pediatric formulation should be dispensed according to instructions in the pharmacy manual. | | | | |
| **BSA- based calculated doses should be administered as per instructions in the pharmacy manual. | | | | |

The Investigator will instruct the patient to take the study treatment as per protocol.

All dosages prescribed and dispensed to the patient and all dose changes during the study must be recorded on the Dosage Administration Record Case Report Form (CRF).

Ruxolitinib will be administered orally twice per day at the assigned starting doses based on age group, given as 5-mg tablets or equivalent dose as oral pediatric formulation. Ruxolitinib (tablet or oral pediatric formulation) should be taken approximately 12 hours apart (morning and night) without regards to food. Ruxolitinib will be administered by hospital personnel in an inpatient setting, or self-administered by the patient in an outpatient setting.

Patients should be instructed not to take study treatment at home on the days of the scheduled pre-dose blood collection. Dosing will be administered after pre-dose blood collection at these visits.

Starting dose as assigned on Day 1 based on age may not be increased even if a patient enters the next subsequent age group during the treatment period.

### Example 4: A Phase II open-label, single-arm, multi-center study of ruxolitinib added to corticosteroids in pediatric subjects with moderate and severe chronic graft vs. host disease after allogeneic stem cell transplantation

### Purpose:

Chronic graft vs. host disease (cGvHD) pathophysiology begins with activation of host antigen-presenting cells (APC) expressed by damaged tissues and/or pathogens (Dhir et al 2014). Activated host APC then present host antigens to donor immune cells, leading to donor T-cell proliferation and inflammatory cytokine production. These inflammatory cytokines then recruit and induce proliferation of additional immune effector cells, thereby perpetuating an adverse cycle of allo reactive tissue injury and inflammation (Paczesny et al 2010). This signaling cascade in cGvHD determined in the mouse model and adult subjects with cGvHD, is expected to be the same in pediatric subjects < 12 years of age as compared to subjects ≥ 12 years of age. Ruxolitinib has been shown to lower pro-inflammatory cytokines in MF patients. In addition, pre-clinical data support the mechanism of action of ruxolitinib in GvHD to: i.) impair APC function, ii.) inhibit donor T cell proliferation, iii.) suppress adverse cytokine production, and iv.) improve survival and disease manifestations in GvHD mouse models (Parampalli Yainanarayana et al 2015) (Heine et al 2013) (Spoerl et al 2014). Published data have shown evidence of clinical efficacy with ruxolitinib treatment when added to immunosuppressive therapy in subjects with SR-cGvHD (Zeiser et al 2015) (Boiko et al 2017).

Clinical studies using ruxolitinib alone or in comparison to best available therapy are currently underway in the SR-cGvHD setting for adult subjects and adolescents ≥ 12 years of age. Despite children being at a lower risk of developing cGvHD than adults (Baird et al 2010), the incidence of cGvHD in the pediatric population is substantial and has increased recently in association with the expanded use of peripheral blood stem cells and unrelated donors (Zecca et al 2002). The treatment of moderate to severe cGvHD in pediatrics is highly variable and mostly extrapolated from the experience in adults. While there is no proven "standard therapy," corticosteroids and calcineurin inhibitors (CNI) are commonly employed as frontline therapy.

Similar to the adults, there are limited treatment options for moderate to severe cGvHD including systemic corticosteroids as initial standard of care for pediatric patients. With only 30% to 50% of the children responding to use of corticosteroids, there is a high unmet medical need for optimal initial and second-line therapies in the pediatric population (Wolff et al 2011). Furthermore, children who respond to initial immunosuppressive treatment, most likely corticosteroids, require it for prolonged periods, consequently having debilitating persistent and irreversible impact on overall health and quality of life (Fraser et al 2006).

Given available data (presented above), in the current setting of a lack of effective first- or second-line treatments for pediatric cGvHD, this study aims to assess the pharmacokinetics, safety and activity of ruxolitinib treatment in pediatric subjects (age ≥ 28 days to < 18 years) with treatment-naive cGvHD or SR-cGvHD. It is expected that ruxolitinib will provide higher rates of disease response compared to steroids +/- Calcineurin Inhibitors (CNI) alone as upfront treatment of moderate to severe cGvHD. It is further expected that this response will be durable during steroid taper, representing a meaningful clinical benefit for pediatric subjects. Expected meaningful clinical benefits among those treated with ruxolitinib include it's steroid sparing effect, reducing the proportion of pediatric subjects experiencing flares during steroid tapering, reducing proportion of subjects with infections and severity of infections, reducing hospitalization duration and requirement for re-admission, and maintenance of graft vs. malignancy effect.

### Objectives and endpoints

**Table 8 Objectives and related endpoints**

| **Objective(s)** | **Endpoint(s)** |
|---|---|
| **Primary objective(s)** | **Endpoint(s) for primary objective(s)** |
| • To evaluate the activity of ruxolitinib added to standard dose corticosteroids +/- CNI in pediatric subjects with moderate or severe treatment naive-cGvHD or SR-cGvHD measured by overall response rate (ORR) at Cycle 7 Day 1 based on all subjects in the study. | • Overall response rate (ORR) at Cycle 7 Day 1, defined as the proportion of subjects demonstrating a complete response (CR) or partial response (PR) without the requirement of additional systemic therapies for an earlier progression, mixed response or non-response. The response is assessed per NIH consensus criteria (Lee et al 2015), and scoring of response will be relative to the organ stage at the start of study treatment. |

| **Secondary objective(s)** | **Endpoint(s) for secondary objective(s)** |
|---|---|
| • To assess pharmacokinetics (PK) of ruxolitinib in treatment-naïve cGvHD and SR-cGvHD pediatric subjects | • Ruxolitinib concentrations by timepoint |
| • To evaluate the safety of ruxolitinib | • Safety and tolerability will be assessed by monitoring the frequency, duration and severity of Adverse Events, including occurrence of any second primary malignancies or infections, and by performing physical exams and evaluating changes in vital signs, tanner stage, chemistry and hematology results from baseline. |
| • To assess duration of response (DOR) | • Duration of response (DOR) is assessed for responders only. DOR is defined as the time from first response until cGvHD progression, death, or the date of addition of systemic therapies for cGvHD. |
| • To estimate ORR at end of Cycle 3 | • Proportion of subjects who achieve OR (CR+PR) at Cycle 4 Day 1 |
| • To assess best overall response (BOR) | • Proportion of subjects who achieved OR (CR+PR) at any time point (until Cycle 7 Day 1 or the start of additional systemic therapy for cGvHD) |
| • To estimate the failure free survival (FFS) | • Composite time to event endpoint incorporating the following FFS events: i) relapse or recurrence of underlying disease or death due to underlying disease, ii) non-relapse mortality, or iii) addition or initiation of another systemic therapy for cGvHD. |
| • To assess cumulative incidence of malignancy relapse/recurrence (MR) | • Malignancy relapse/recurrence (MR) is defined as the time from date of treatment assignment to hematologic malignancy relapse/recurrence. Calculated for subjects with underlying hematologic malignant disease. |
| • To assess non-relapse mortality (NRM) | • Non-relapse mortality (NRM), defined as the time from date of treatment assignment to date of death not preceded by underlying disease relapse/recurrence. |
| • To assess overall survival (OS) | • Overall survival, defined as the time from the date of treatment assignment to the date of death due to any cause. |
| • To assess a reduction of at least ≥ 50% in daily corticosteroid use at Cycle 7 Day 1 | • Proportion of subjects with ≥ 50% reduction from baseline in daily corticosteroid dose at Cycle 7 Day 1 |
| • To assess a reduction to a low dose corticosteroid dose at Cycle 7 Day 1 | • Proportion of subjects with reduction from baseline in daily corticosteroid dose to ≤ 0.2 mg/kg/day methylprednisolone (or equivalent dose of ≤ 0.25 mg/kg/day prednisone or prednisolone) at Cycle 7 Day 1 |
| • To assess graft failure | • Assess using donor cell chimerism, defined as initial whole blood or marrow donor chimerism for those who had ≥ 5% donor cell chimerism at baseline. If donor cell chimerism declines to < 5% on subsequent measurements, the graft failure is declared. |

### Study design

This open-label, single-arm, Phase II multi-center study will investigate the activity, pharmacokinetics and safety of ruxolitinib added to the subject's immunosuppressive regimen among infants, children, and adolescents aged ≥ 28 days to < 18 years old with either moderate to severe treatment-naive cGvHD or SR-cGvHD. Approximately 42 subjects will be enrolled in this study. Subjects will be grouped according to their age as follows: Group 1 includes subjects ≥ 12y to < 18y, Group 2 includes subjects ≥ 6y to <12 y, Group 3 includes subjects ≥ 2y to < 6y, and Group 4 includes subjects ≥ 28days to < 2y. Subjects will remain in the age group throughout the study based on the age at the time of start of treatment. Enrollment initiation into the youngest age group, Group 4, will be subject to the availability of data in this age group from study in Example 3, as well as a review of available PK, safety, and activity data generated from Groups 1 to 3 in the current study, in consultation with the data monitoring committee (DMC) and a final decision by the Sponsor. At least 5 evaluable subjects per Group are needed for the primary analysis in Groups 1, 2 and 3. No minimum number of evaluable subjects are needed in Group 4.

After a screening period from Day -28 to Day -1, eligible subjects will begin the investigational treatment (ruxolitinib) on Cycle 1 Day 1 and will be treated for up to a maximum of 3 years (39 cycles/week 156) or until early discontinuation. Subjects who discontinue ruxolitinib for any reason earlier than 39 cycles will be followed every 6 months until 3 years from their first dose of ruxolitinib is reached. All subjects continuing to receive ruxolitinib treatment benefit following 3 years of treatment will be given the possibility to continue ruxolitinib outside the study, from another source, where permitted in accordance to local laws and regulations.

The ruxolitinib dose is based on the preliminary efficacy and safety data generated with this dose in subjects with steroid-refractory graft vs. host disease (SR-GvHD) (Zeiser et al 2015), and based on PK/safety data generated from the Phase III trials CRUXOLITINIBC2301 and CRUXOLITINIBD2301. As subjects ≥ 12 y to < 18 y are already being treated with 10 mg BID in CRUXOLITINIBD2301, this dose is the recommended phase II dose (RP2D), and will be used to treat all subjects in this age group.

Pediatric subjects enrolled in the pediatric aGvHD study in Example 3 will provide PK data that will be used to confirm the adequacy of the doses described above and thus patients < 12 years will only be enrolled in Groups 2 to 4 in the current study once the dose is confirmed in the appropriate age group. If a different dose is confirmed in the pediatric aGvHD study in Example 3, the dose will need to be adjusted accordingly in the current study. Subjects enrolled in the adult aGvHD study and cGvHD study may also provide PK data that will be used as additional information to confirm adequacy of this dose.

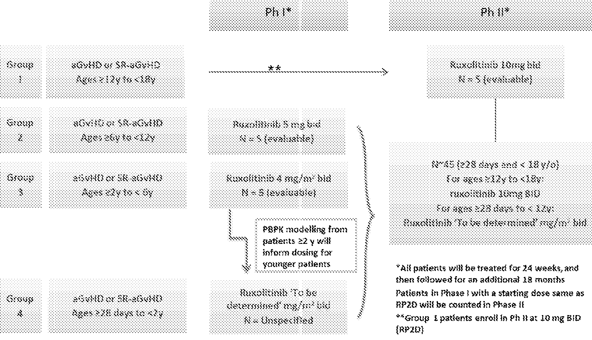

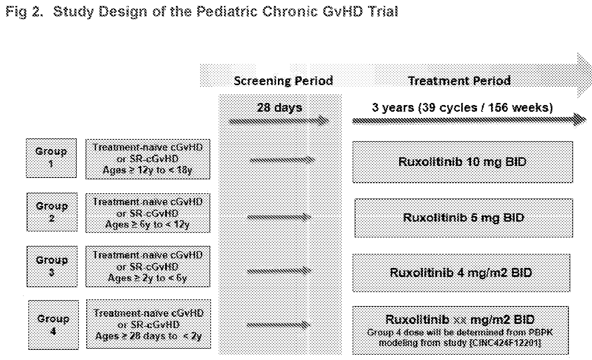

The Study Design for this trial is illustrated by Fig. 2.

### Inclusion Criteria

Subjects eligible for inclusion in this study must meet all of the following criteria:
1. Male or female subjects age ≥ 28 days and < 18 years at the time of informed consent.
2. Subjects who have undergone a successful alloSCT from any donor source (matched unrelated donor, sibling, haplo-identical) using bone marrow, peripheral blood stem cells, or cord blood. Recipients of myeloablative or reduced intensity conditioning are eligible.
3. Subjects with diagnosed moderate to severe cGvHD according to NIH 2014 Consensus Criteria prior to Cycle 1 Day 1. Other possible diagnoses for clinical symptoms supporting the cGvHD diagnosis must be excluded (e.g. infection, drug side effects, malignancy). Subjects must be either:
   - Treatment-naive cGvHD subjects that have not received any prior systemic treatment for cGvHD except for a maximum 72h of prior systemic corticosteroid therapy of methylprednisolone or equivalent after the onset of chronic GvHD. Subjects are allowed to have received prior systemic treatment for cGvHD prophylaxis (as long as the prophylaxis was started prior to the diagnosis of cGvHD).
      OR
   - Steroid-refractory moderate to severe cGvHD as per institutional criteria, and still receiving systemic corticosteroids for the treatment of cGvHD for a duration of <18 months prior to Cycle 1 Day 1. In case the corticosteroids were interrupted due to response, the duration of < 18 months applies to the last period of corticosteroids use.
4. Able to swallow study medication.
5. Written study informed consent (and assent if appropriate) from the subject and/or parent/legal guardian

### Exclusion criteria

Subjects meeting any of the following criteria are not eligible for inclusion in this study.
1. SR-cGvHD subjects with a prior cGvHD treatment with a JAK1- or a JAK2- or a JAK1/2-inhibitor are not allowed, except when the subject achieved complete or partial response and has been off JAK inhibitor treatment for at least 4 weeks prior to Cycle 1 Day 1, or up to 5 times the half-life of the prior JAK inhibitor, whichever is longer.
2. Subjects who initiated systemic calcineurin inhibitors (CNI; cyclosporine or tacrolimus) within 3 weeks prior to the start of ruxolitinib on Cycle 1 Day 1. Note: Systemic CNI are allowed when initiated > 3 weeks from start of ruxolitinib.
3. Failed prior alloSCT within the past 6 months; subjects with relapsed primary malignancy, or who have been treated for relapse after the alloSCT was performed, or who require withdrawal of immune suppression as pre-emergent treatment of early malignancy relapse.
4. Significant respiratory disease including subjects who are on mechanical ventilation or who have a resting oxygen saturation < 90% by pulse-oximetry on room-air.
5. Impairment of gastrointestinal (GI) function (unrelated to GvHD) or GI disease (unrelated to GvHD) that may significantly alter the absorption of oral ruxolitinib (e.g., ulcerative diseases, uncontrolled nausea, vomiting, diarrhea, malabsorption syndrome or small bowel resection).
6. Cholestatic disorders, or unresolved sinusoidal obstructive syndrome/veno-occlusive disease of the liver (defined as persistent bilirubin abnormalities not attributable to cGvHD and ongoing organ dysfunction).
7. Presence of clinically active uncontrolled infection including significant bacterial, fungal, viral or parasitic infection requiring treatment. Infections are considered controlled if appropriate therapy has been instituted and, at the time of screening, no signs of progression are present. Progression of infection is defined as hemodynamic instability attributable to sepsis, new symptoms, worsening physical signs or radiographic findings attributable to infection. Persisting fever without other signs or symptoms will not be interpreted as progressing infection.
8. Known human immunodeficiency virus (HIV) infection.
9. Evidence of uncontrolled hepatitis B virus (HBV) or hepatitis C virus (HCV) based on assessment done by Investigator or delegate.
10. cGvHD occurring after a non-scheduled donor lymphocyte infusion (DLI) administered for pre-emptive treatment of malignancy recurrence. Subjects who have received a scheduled DLI as part of their transplant procedure and not for management of malignancy relapse are eligible.
11. Any condition that would, in the investigator's judgment, interfere with full participation in the study, including administration of study drug and attending required study visits; pose a significant risk to the subject; or interfere with interpretation of study data.
12. Known allergies, hypersensitivity, or intolerance to any of the study medications, excipients, or similar compounds.
13. History of bone disorders such as osteogenesis imperfecta, rickets, renal osteodystrophy, osteomyelitis, osteopenia, fibrous dysplasia, osteomalacia etc. prior to the underlying diagnosis which resulted in the alloSCT.
14. History of endocrine or kidney related growth retardation prior to the underlying diagnosis which resulted in the alloSCT.
15. Female adolescent subjects who are pregnant or breast feeding.
16. Female subjects of childbearing potential (e.g., are menstruating) who do not agree to abstinence, or, if sexually active, do not agree to use highly effective contraception.
17. Evidence of clinically active tuberculosis (clinical diagnosis per local practice)
18. Any corticosteroid therapy for indications other than cGvHD at doses > 1 mg/kg/daymethylprednisolone (or equivalent prednisone dose 1.25 mg/kg/day) within 7 days of the screening visit.
19. Current therapy with medications that interfere with coagulation or platelet functionincluding but not limited to aspirin and related drugs, heparin, and warfarin (to minimize risk of bleeding). Note: Heparin or Low Molecular Weight Heparin (LMWH) is allowed if used at sub-therapeutic dose for e.g., prophylaxis of sinusoidal obstructive syndrome/veno-occlusive disease of the liver.
20. Subject is receiving fluconazole at daily doses higher than 200mg.
21. Subject is receiving and does not agree to stop herbal preparations/medications. These herbal medications include, but are not limited to, St. John's Wort, Kava, ephedra (ma huang), gingko biloba, dehydroepiandrosterone (DHEA), yohimbe, saw palmetto, and ginseng. Subjects must stop using herbal medications at least 7 days prior to first dose of study treatment.
22. History of progressive multifocal leuko-encephalopathy (PML).
23. Investigational treatment within 30 days prior to treatment initiation or within 5 half-lives of the investigational product, whichever is greater.
24. Presence of severely impaired renal function (confirmed within 72h prior to ruxolitinib start) defined by:
   - Glomerular Filtration Rate (GFR) < 30 mL/min/1.73 m2, using estimated creatinine clearance calculated by updated bedside Schwartz equation or Cockroft Gault equation,
      or
   - renal dialysis requirement
25. Serious comorbid diseases as per investigator judgement
26. Life expectancy of less than 1 month as per investigator judgement.
27. Clinically significant or uncontrolled cardiac disease, including any of the following:
   - Acute myocardial infarction within 6 months from Cycle 1 Day 1 ruxolitinib administration
   - Uncontrolled hypertension
   - New York Heart Association Class III or IV congestive heart failure
   - Unstable angina within last 6 months from screening
   - Clinically significant (symptomatic) cardiac arrhythmias (e.g., sustained ventricular tachycardia, and clinically significant second or third degree AV block without a pacemaker, circulatory collapse requiring vasopressor or inotropic support, or arrhythmia requiring therapy).

### Treatment

Study treatment includes:
- Investigational treatment:
- Refers to ruxolitinib (investigational drug)
- Other study treatment:
- Refers to the concomitant use of corticosteroids to treat treatment-naive cGvHD or SR-cGvHD

Ruxolitinib will be administered as a 5 mg ruxolitinib tablet or as ruxolitinib oral pediatric formulation twice a day.
- Treatment-naive cGvHD: In addition to ruxolitinib, treatment must include methylprednisolone (or equivalent prednisone)
- SR-cGvHD: In addition to ruxolitinib, concomitant use of corticosteroids is allowed.

In addition to study treatment, subjects may receive standard alloSCT supportive care including anti-infective medications and transfusion support.

Systemic immunosuppressive medications used for the prophylaxis of cGvHD may be continued after Cycle 1 Day 1 if initiated prior to diagnosis of cGvHD. Systemic CNIs may be continued after Cycle 1 Day 1 if initiated at least 3 weeks prior to the start of ruxolitinib, i.e. at Cycle 1 Day 1. CNI should be used at standard dosing and adjusted to therapeutic trough levels. Continued use of topical corticosteroid therapy for cGvHD per institutional guidelines are permitted. For SR-cGvHD subjects, cessation of other systemic treatment for cGvHD other than corticosteroids +/- CNI will be required prior to ruxolitinib initiation.

**Table 9 Dose and treatment schedule**

| **Investigational treatment** | **Age groups** | **Pharmaceutical form and Route of Administration** | **Dose** | **Frequency and/or Regimen** |
|---|---|---|---|---|
| Ruxolitinib | Group 1 | 5 mg tablet* for oral use OR oral pediatric formulation of Example 1 | 10 mg BID (2 tablets orally BID) OR 10 mg BID oral pediatric formulation of Example 1** | Twice per day |
| | ≥ 12 years old to < 18 years old | | | |
| Ruxolitinib | Group 2 | 5 mg tablet* for oral use OR oral pediatric formulation* of Example 1 | 5 mg BID (1 tablet orally BID) OR 5 mg BID oral pediatric formulation of Example 1** | Twice per day |
| | ≥ 6 years old to < 12 years old | | | |
| Ruxolitinib | Group 3 | 5 mg tablet* for oral use OR oral pediatric formulation of Example 1 | 4 mg/m² BID (either tablet orally OR oral pediatric formulation of Example 1)** | Twice per day |
| | ≥ 2 years old to < 6 years old | | | |
| Ruxolitinib | Group 4 | oral pediatric formulation of Example 1 | X mg/m² BID (oral pediatric formulation of Example 1)** | Twice per day |
| | ≥ 28 days to < 2 years old | | | |
| | | | To be defined from study CRUXOLITINIBF 12201 | |

| | | | | |
|---|---|---|---|---|
| *Tablet for oral use may be crushed as per instructions in the pharmacy manual (if calculated dose based on BSA is not 5 mg or 10 mg, then crushing is not permitted. In this case, oral pediatric formulation should be administered). Oral pediatric formulation should be dispensed according to instructions in the pharmacy manual. **Calculated doses may be rounded to the nearest available volume as per instructions in the pharmacy manual | | | | |

The Investigator will instruct the subject to take the investigational treatment as per protocol.

All dosages prescribed and dispensed to the subject and all dose changes during the study must be recorded on the Dosage Administration Record electronic case report form (CRF).

Ruxolitinib will be administered orally twice per day at the assigned dose based on age group, given as 5 mg tablets or equivalent dose as oral pediatric formulation. Ruxolitinib (tablet or oral pediatric formulation) should be taken approximately 12 hours apart (morning and night) without regards to food. Ruxolitinib will be administered by Investigator or delegate, or self-administered by the subject or parent/legal guardian in an outpatient setting.

Subjects should be instructed not to take ruxolitinib at home on the days of the scheduled pre-dose blood collection for PK and biomarker samples. Dosing will be administered after pre-dose blood collection at these visits.

The ruxolitinib dose as assigned on Cycle 1 Day 1 (based on age) must not be changed until the subject completes the Cycle 7 Day 1 visit assessments, unless dose adjustments are needed to manage safety events. After Cycle 7 Day 1, the Investigator must re-evaluate the assigned ruxolitinib dose based on the subject's age and/or growth. Dosing based on growth should be adjusted if the newly calculated dose is a > 10% change from the previously calculated dose. All dose changes must be recorded on the Dosage Administration Record electronic case report form (eCRF).

### Example 5: Stable Pediatric Oral Solution Formulation at 1mg/mL concentration

**Table 10. Composition of the formulation**

| **Component** | **Functionality** | **Amount per unit (mg/mL)** |
|---|---|---|
| Ruxolitinib phosphate* | Active ingredient | 1.320 |
| Propylene glycol | Co-solvent | 150.000 |
| Methyl paraben | Preservative | 1.200 |
| Propyl paraben | Preservative | 0.400 |
| Sucralose | Sweetener | 2.000 |
| Citric acid | pH-adjusting agent | 4.270 |
| Strawberry flavor | Flavor | 2.500 |
| Purified water | Vehicle | 853.310 |
| Total | | q.s to 1mL |

| | | |
|---|---|---|
| * salt factor 1.320 | | |

### Example 6: Stablility Results

### A. Objections

This example contains stability data for:
Technical batches variant 001 stability data of ruxolitinib 1mg/mL and 5mg/mL solution covering storage period up to 6 months, and technical batches variant 002 stability data of ruxolitinib 5mg/mL (891147, 891148) for up to 24 months.

Clinical batch variant 002 stability data of ruxolitinib 5mg/mL solution (891147) covering storage period up to 12months data, in-use study 42 days and freeze and thaw study covering days up to 28 days.

The technical batches studied are tabulated in Table 14 and the clinical batch studies are tabulated in Table 28. Ruxolitinib is a BCS class I compound. Current solution formulation is an RUXOLITINIB water base solution with common preservatives and flavors with / without sucrose.

The development stability report DSR2810 (5.0) applies to RUXOLITINIB Img/mL and 5mg/mL solution formulation technical batches and clinical batches.

**Table 11. Description of drug product variants**

| | **Product Code ¹** | | |
|---|---|---|---|
| **Dosage strength** | **Basis number** | **Variant number** | **Packaging/Comment** |
| 1 mg/mL sugar formulation | 7010281 | 001 | BO_125 AMBER_CR_CLOSURE |
| 1 mg/mL non-sugar formulation | n.a² | n.a² | BO_125 AMBER_CR_CLOSURE |
| 1 mg/mL sugar formulation | 7010282 | 001 | BO_300BR |
| 1 mg/mL non-sugar formulation | n.a² | n.a² | BO_300BR |
| 5 mg/mL sugar formulation | 7010279 | 001 | BO_125 AMBER_CR_CLOSURE |
| 5 mg/mL non-sugar formulation | n.a² | n.a² | BO_125 AMBER_CR_CLOSURE |
| 5 mg/mL sugar formulation | 7010280 | 001 | BO_300BR |
| 5 mg/mL non-sugar formulation | n.a² | n.a² | BO_300BR |
| Placebo (1 mg/mL) non-sugar formulation | n.a² | n.a² | BO_125 AMBER_CR_CLOSURE |
| Placebo (5 mg/mL) sugar formulation | n.a² | n.a² | BO_125 AMBER_CR_CLOSURE |
| 5 mg/mL 2034827(140mL) | 7010280 | 002 | BO_300 AMBER_CR_CLOSURE |
| 5 mg/mL H0004(60ml) | 7010279 | 002 | BO_125_AMBER_CR_CLOSURE |
| 5 mg/mL H0004(140ml) | 7010280 | 002 | BO_300_AMBER_CR_CLOSURE |

| | | | |
|---|---|---|---|
| ¹ Novartis internal reference system ² Not applicable | | | |

Based on the stability data obtained, the sugar formulation is considered to be stable during clinical development when packed and stored as described in Table 12. For justifications refer to Table 33.

**Table 02. Description of shelf life, storage conditions, packaging and transport category and in-use period**

| **Dosage strength(s)** | | **1mg/mL Clinical supplies** |
|---|---|---|
| Product code(s) | | |
| | Shelf life(1mg/mL) | |
| 7010282.001 | | 21 months |
| 7010281.001 | | 18 months |
| Packaging | | BO_125_AMBER_CR_CLOSURE and BO_300BR |
| Storage requirements | | Store at 2-8°C, do not freeze |
| Transport category | | FRIGO, Do not freeze |
| In-use period | | Not available |
| Packaging category | | STANDARD |
| In-use storage requirements | | Not available |

| **Dosage strength(s)** | | **5mg/mL Clinical supplies** |
|---|---|---|
| Product code(s) Shelf life(5mg/mL) | | |
| 7010279.002 (891148), 7010280.002 (891147) | | 24 months |
| 7010279.001, 7010280.001 | | To be determined |
| Packaging | | BO_125_AMBER_CR_CLOSURE and BO_300BR |
| Storage requirements | | Do not store above 25°C |
| Transport category | | TEMPCONTROL |
| In-use period | | |
| 7010279.002 (891148), 7010280.002 (891147) | | 42 days |
| 7010279.001, 7010280.001 | To be determined | |
| Packaging category | STANDARD | |
| In-use storage requirements | Do not store above 25°C | |

| **Dosage strength(s)** | **1mg/mL Bulk** | |
|---|---|---|
| Product code(s) | | |
| Shelf life(1mg/mL) 7010282.001 | 21 months | |
| 7010281.001 | 18 months | |
| Packaging | BO_125_AMBER_CR_CLOSURE and BO_300BR | |
| Storage requirements | Store at 2-8°C, Do not freeze | |
| Transport category | FRIGO, Do not freeze | |
| Packaging category | Not available | |

| **Dosage strength(s)** | **5mg/mL Bulk** | |
|---|---|---|
| Product code(s) Shelf life (5mg/mL) | | |
| 7010279.002(891148) | 24 months | |
| 7010280.002(891147) | 24 months | |
| 7010279.001, 7010280.001 | To be determined | |
| Packaging | BO_125_AMBER_CR_CLOSURE and BO_300BR | |
| Storage requirements | Do not store above 25°C | |
| Transport category | TEMPCONTROL | |
| Packaging category | STANDARD | |

### B. Specifications of drug product

The analytical specifications are laid down in the current specifications for clinical development AS2810.

### C. Stability studies

### Description of packaging

**Table 13. Description of packaging**

| **Abbreviation** | **Description** |
|---|---|
| BO_125 AMBER_CR_CLOSURE | 125 mL amber glass bottle with child resistant closure. |
| BO_300BR | 300 mL brown glass bottle closed with a polypropylene child resistant closure. |
| BO_300BR AMBER_CR_CLOSURE | 300 mL amber glass bottle with child resistant closure. |

### Batches tested and stability program

**Table 14. Description of batches tested and stability program for technical batches**

| **Strength Batch** | **Product code⁶** | **Batch type** | **Batch size [units]** | **Primary packaging** | **Date of manufactur e** | **Site of manufacture** | **DS batch** | **DS variant** |
|---|---|---|---|---|---|---|---|---|
| 1mg/mL (Placebo solution) T010 0216 ¹ | n.a | technical | n.a | BO_125_AMBER_ CR_CLOSURE | n.a | Novartis Pharma AG, India / IN | n.a | n.a |
| 5mg/mL (Placebo solution) T011 0216 ² | n.a | technical | n.a | BO_125_AMBER_ CR_CLOSURE | n.a | Novartis Pharma AG, India / IN | n.a | n.a |
| 1mg/mL (solution) T109 1215³ | n.a | technical | 15 liters | BO_125_AMBER_ CR_CLOSURE | 30-Oct-2015 | Novartis Pharma AG, India / IN | 1010002736 | RUXOLITI NIB AZA.009 |
| 1mg/mL (solution) T111 1215⁴ | n.a | technical | 15 liters | BO_125_AMBER_ CR_CLOSURE | 11-Jan-2016 | Novartis Pharma AG, India / IN | 1010002736 | RUXOLITI NIB AZA.009 |
| 5mg/mL (solution) T110 1215³ | n.a | technical | 15 liters | BO_125_AMBER_ CR_CLOSURE | 06-Jan-2016 | Novartis Pharma AG, India / IN | 1010002736 | RUXOLITI NIB AZA.009 |
| 5mg/mL (solution) T112 1215⁴ | n.a | technical | 15 liters | BO_125_AMBER_ CR_CLOSURE | 04-Jan-2016 | Novartis Pharma AG, India / IN | 1010002736 | RUXOLITI NIB AZA.009 |
| 1mg/mL (solution) T109 1215³ | n.a | technical | 15 liters | BO_300BR | 30-Oct-2015 | Novartis Pharma AG, India / IN | 1010002736 | RUXOLITI NIB AZA.009 |
| 1mg/mL (solution) T111 1215⁴ | n.a | technical | 15 liters | BO_300BR | 11-Jan-2016 | Novartis Pharma AG, India / IN | 1010002736 | RUXOLITI NIB AZA.009 |
| 5mg/mL (solution) T110 1215³ | n.a | technical | 15 liters | BO_300BR | 06-Jan-2016 | Novartis Pharma AG, India / IN | 1010002736 | RUXOLITI NIB AZA.009 |
| 5mg/mL (solution) T112 1215⁴ | n.a | technical | 15 liters | BO_300BR | 04-Jan-2016 | Novartis Pharma AG, India / IN | 1010002736 | RUXOLITI NIB AZA.009 |
| 5mg/mL (solution) H0004 (60mL)⁵ | 7010279. 002 | technical | 200 liters | BO_125_AMBER_ CR_CLOSURE | 12 Oct 2018 | Delpharm Huningue SAS, France | 1010002726 | RUXOLITI NIB AZA.006 |
| 5mg/mL (solution) H0004 (140mL) ⁵ | 7010280. 002 | technical | 200 liters | BO_300_AMBER_ CR_CLOSURE | 12 Oct 2018 | Delpharm Huningue SAS, France | 1010002726 | RUXOLITI NIB AZA.006 |

| | | | | | | | | |
|---|---|---|---|---|---|---|---|---|
| n.a Not applicable ¹ Without sucrose formulation ² With sucrose formulation ³ Without sucrose formulation ⁴ With sucrose formulation ⁵ With propylene glyocol, methyl paraben and propyl paraben ⁶ Novartis internal reference system | | | | | | | | |

**Table 15. Stability program 1mg/mL Placebo solution, T010 0216,**

| *BO_125_AMBER_CR_CLOSURE* | |
|---|---|
| **Storage condition** | **Test intervals¹** |
| 5°C/Ambient RH | **1, 3, 6,** 9, 12, 18, 24 |
| 25°C/60% RH | **1, 3, 6**, 9, 12, 18, 24 |
| 40°C/75% RH | **1, 3, 6** |

| | |
|---|---|
| ¹ The tested pull points are shown in bold. | |

**Table 16. Stability program 5mg/mL Placebo solution, T011 0216,**

| *BO_125_AMBER_CR_CLOSURE* | |
|---|---|
| **Storage condition** | **Test intervals¹** |
| 5°C/Ambient RH | **1, 3, 6**, 9, 12, 18, 24 |
| 25°C/60% RH | **1, 3, 6**, 9, 12, 18, 24 |
| 40°C/75% RH | **1, 3, 6** |

| | |
|---|---|
| ¹ The tested pull points are shown in bold | |

**Table 17. Stability program 1mg/mL solution, T109 1215, BO_125_AMBER_CR_CLOSURE**

| **Storage condition** | **Test intervals¹** |
|---|---|
| 5°C/Ambient RH | **1, 3, 6**, 9, 12, 18, 24 |
| 25°C/60% RH | **1, 3, 6,** 9, 12, 18, 24 |
| 40°C/75% RH | **1, 3, 6** |

| | |
|---|---|
| ¹ The tested pull points are shown in bold | |

**Table18. Stability program 1mg/mL solution, T111 1215, BO_125_AMBER_CR_CLOSURE**

| **Storage condition** | **Test intervals¹** |
|---|---|
| 5°C/Ambient RH | **1, 3, 6**, 9, 12, 18, 24 |
| 25°C/60% RH | **1, 3, 6,** 9, 12, 18, 24 |
| 40°C/75% RH | **1, 3, 6** |

| | |
|---|---|
| ¹ The tested pull points are shown in bold | |

**Table 19. Stability program 5mg/mL solution, T110 1215, BO_125_AMBER_CR_CLOSURE**

| **Storage condition** | **Test intervals¹** |
|---|---|
| 5°C/Ambient RH | **1, 3, 6**, 9, 12, 18, 24 |
| 25°C/60% RH | **1, 3, 6**, 9, 12, 18, 24 |
| 40°C/75% RH | **1, 3, 6** |

| | |
|---|---|
| ¹ The tested pull points are shown in bold | |

**Table 20. Stability program 5mg/mL solution, T112 1215, BO_125_AMBER_CR_CLOSURE**

| **Storage condition** | **Test intervals¹** |
|---|---|
| 5°C/Ambient RH | **1, 3, 6**, 9, 12, 18, 24 |
| 25°C/60% RH | **1, 3, 6**, 9, 12, 18, 24 |
| 40°C/75% RH | **1, 3, 6** |

| | |
|---|---|
| ¹ The tested pull points are shown in bold | |

**Table 21. Stability program 1mg/mL solution, T109 1215, BO_300BR**

| **Storage condition** | **Test intervals¹** |
|---|---|
| 5°C/Ambient RH | **1, 3, 6**, 9, 12, 18, 24 |
| 25°C/60% RH | **1, 3, 6**, 9, 12, 18, 24 |
| 40°C/75% RH | **1, 3, 6** |

| | |
|---|---|
| ¹ The tested pull points are shown in bold | |

**Table 22. Stability program 1mg/mL solution, T111 1215, BO_300BR**

| **Storage condition** | **Test intervals¹** |
|---|---|
| 5°C/Ambient RH | **1, 3, 6,** 9, 12, 18, 24 |
| 25°C/60% RH | **1, 3, 6**, 9, 12, 18, 24 |
| 40°C/75% RH | **1, 3, 6** |

| | |
|---|---|
| ¹ The tested pull points are shown in bold | |

**Table 23. Stability program 5mg/mL solution, T110 1215, BO_300BR**

| **Storage condition** | **Test intervals¹** |
|---|---|
| 5°C/Ambient RH | **1, 3, 6**, 9, 12, 18, 24 |
| 25°C/60% RH | **1, 3, 6**, 9, 12, 18, 24 |
| 40°C/75% RH | **1, 3, 6** |

| | |
|---|---|
| ¹ The tested pull points are shown in bold | |

**Table 24. Stability program 5mg/mL solution, T112 1215, BO_300BR**

| **Storage condition** | **Test intervals¹** |
|---|---|
| | |
| 5°C/Ambient RH | **1, 3, 6**, 9, 12, 18, 24 |
| 25°C/60% RH | **1, 3, 6**, 9, 12, 18, 24 |
| 40°C/75% RH | **1, 3, 6** |

| | |
|---|---|
| ¹ The tested pull points are shown in bold | |

**Table 25. Stability program 5mg/mL solution, H0004 (60mL), BO_125_AMBER_CR_CLOSURE**

| **Storage condition** | **Test intervals¹** |
|---|---|
| 5°C/Ambient RH | **Initial, 45², 3, 6, 9, 12, 18, 24**,36 |
| 25°C/60% RH | **Initial, 45² 3, 6, 9, 12, 18, 24**,36 |
| 40°C/75% RH | **Initial, 45² 3, 6** |

| | |
|---|---|
| ¹ The tested pull points are shown in bold. ² 45days | |

**Table 26. Stability program 5mg/mL solution, H0004 (140mL),**

| *BO_300_AMBER_CR_CLOSURE* | |
|---|---|
| **Storage condition** | **Test intervals¹** |
| 5°C/Ambient RH | **Initial, 45² 3, 6, 9, 12, 18, 24**,36 |
| 25°C/60% RH | **Initial, 45² 3, 6, 9, 12, 18, 24**,36 |
| 40°C/75% RH | **Initial, 45² 3, 6** |

| | |
|---|---|
| ¹ The tested pull points are shown in bold. ² 45 days | |

**Table 27. Stability program 5mg/mL solution, H0004 (60mL), In-Use study**

| *BO_125_AMBER_CR_CLOSURE* | |
|---|---|
| **Storage condition** | **Test intervals¹** |
| 25°C/60% RH | **30days, 42days** |

| | |
|---|---|
| ¹ The tested pull points are shown in bold. | |

**Table 28. Description of batches tested and stability program for clinical batch**

| **Strength Batch** | **Product code¹** | **Batch type** | **Batch size [units]** | **Primary packaging** | **Date of manufacture** | **Site of manufacture** | **DS batch** | **DS variant** |
|---|---|---|---|---|---|---|---|---|
| 5mg/mL (solution) 2034827 (140mL) | 7010280.002 | clinical | 1335 | BO_300_AMBER_CR _CLOSURE | 31 Jul 2019 | Delpharm Huningue SAS, France | R0004 | RUXOLITINIB AZA.013 |

**Table 29. Stability program 5mg/mL solution, 2034827 (140mL), In-Use study**

| *BO_300_AMBER_CR_CLOSURE* | |
|---|---|
| **Storage condition** | **Test intervals¹** |
| 25°C/60% RH | **30days, 42days** |
| ¹ The tested pull points are shown in bold. | |

**Table C0. Stability program 5mg/mL solution, 2034827 (140mL), Freeze and thaw study**

| *BO_300_AMBER_CR_CLOSURE* | |
|---|---|
| **Storage condition** | **Test intervals¹** |
| -20°C/ambient RH / 25°C/60% RH 28 days | **28** days |
| ¹ The tested pull points are shown in bold. | |

**Table 31. Stability program 5mg/mL solution, 2034827**

| *(140mL)_Upright_BO_300_AMBER_CR_CLOSURE* | |
|---|---|
| **Storage condition** | **Test intervals¹** |
| 5°C/Ambient RH | **6W² 3, 6, 9, 12,** 18, 24,36 |
| 25°C/60% RH | **6W² 3, 6, 9, 12,** 18, 24,36 |
| 30°C/75% RH | **3, 6, 9, 12** |
| 40°C/75% RH | **6W² 3, 6** |

| | |
|---|---|
| ¹The tested pull points are shown in bold. ² 6Weeks | |

**Table C2. Stability program 5mg/mL solution, 2034827 (140mL)_**

| *Inverted_BO_300_AMBER_CR_CLOSURE* | |
|---|---|
| **Storage condition** | **Test intervals¹** |
| 5°C/Ambient RH | **6W² ,3, 6, 9, 12,** 18, 24,36 |

| **Storage condition** | **Test intervals¹** |
|---|---|
| 25°C/60% RH | **3, 6, 9, 12,** 18, 24,36 |
| 30°C/75% RH | **3, 6, 9, 12** |
| 40°C/75% RH | **6W² ,3, 6** |

| | |
|---|---|
| ¹The tested pull points are shown in bold. ² 6Weeks | |

### Special test

### Freeze and thaw cycle test

This test was performed with one clinical batch (5mg/mL, batch: 2034827) packaged in BO_300_AMBER_CR_CLOSURE container. The stability samples were stored for four complete freeze and thaw cycles (-20°C/ambient RH for 6 days, followed by 1 day at 25°C/60% RH). Samples were taken after 28 days and analyzed. The results for the freeze and thaw cycle test for 5mg/mL (140mL) BO_300_AMBER_CR_CLOSURE container is considered the worst case (with the larger headspace) and will also apply to the 5mg/mL (60mL) BO_125_AMBER_CR_CLOSURE container.

### In-use study

This test was performed with one technical batch (5mg/mL, batch H0004) packaged in BO_125_AMBER_CR_CLOSURE and one clinical batch (5mg/mL, batch 2034827) packaged in BO_300_AMBER_CR_CLOSURE container.

Both the technical and clinical batches were stored at 25°C/60% RH up to 42 days and analyzed.

### D. Results, discussion and interpretation

### Technical batches

### Testing at 5 degree C/ambient RH

### BO_125_AMBER_CR_CLOSURE and BO_300_AMBER_CR_CLOSURE (5mg/mL dosage)

All the results obtained for technical batch no: H0004 (60mL)/ (5mg/mL) and H0004 (140mL)/ (5mg/mL) BO_125_AMBER_CR_CLOSURE and BO_300_AMBER_CR_CLOSURE respectively are well within specifications. There was no significant change in pH values, enantiomer content, degradation products and assay values of RLTXOLITINIB, methyl paraben and propyl paraben for the formulation (5 mg/mL) at 5°C/ambient RH.

There is no significant change on all testing physical and chemical properties, which included assay of active and preservatives, degradation products, enantiomer, pH, and appearance up to 24 months.

### Long term condition at 25 degree C/60 percent RH

### BO_125_AMBER_CR_CLOSURE and BO_300_AMBER_CR_CLOSURE (5mg/mL dosage)

All the results obtained for technical batch no: H0004 (60mL)/(5mg/mL) and H0004 (140mL)/ (5mg/mL)BO_125_AMBER_CR_CLOSURE and BO_300_AMBER_CR_CLOSURE respectively are well within specifications. There was no significant change in pH values, enantiomer content, degradation products and assay values of RUXOLITINIB, methyl paraben and propyl paraben for the formulation (5 mg/mL) at long term condition 25°C/60% RH.

There is no significant change on all testing physical and chemical properties, which included assay of active and preservatives, degradation products, enantiomer, pH, and appearance up to 24 months.

### Testing at 40 degree C/75 percent RH

### BO_125_AMBER_CR_CLOSURE and BO_300_AMBER_CR_CLOSURE (5mg/mL dosage)

All the results obtained for technical batch no: H0004 (60mL)/ (5mg/mL) and H0004 (140mL)/ (5mg/mL) BO_125_AMBER_CR_CLOSURE and BO_300_AMBER_CR_CLOSURE respectively there was no significant change in pH values, enantiomer content and assay values of RUXOLITINIB, methyl paraben and propyl paraben for the formulation (5 mg/mL) at accelerated condition 40°C/75% RH.

For both batches H0004 (60mL) and H0004 (140mL) in 6month analysis OOS results were observed for degradation products. For batch no: H0004 (60mL) and H0004 (140mL) an unspecified impurity RRT 0.33 at 0.6% and 0.7% level results were observed respectively. During root cause investigation it was determined to be preservative related degradation. For more details refer AQWA#2184230. Also, for 6 months analysis of both H0004 (60mL) and H0004 (140mL) the 536-11 degradation product was observed at 0.4% level respectively. Although these do not exceed the early phase limits set in the current specification, the increase needs to be monitored.

All other results (physical and chemical) remained within the specification limits for these batches.

### In-use study

### BO_125_AMBER_CR_CLOSURE (5mg/mL dosage)

This test was performed with one technical batch: H0004 (60mL) packaged in BO_125_AMBER_CR_CLOSURE container. Samples were stored at 25°C/60% RH up to 42 days in open bottles and analyzed.

There is no significant change on all testing physical and chemical properties, which included assay of active and preservatives, degradation products, enantiomer, pH, and appearance at 42 days.

### Clinical batch

### Testing at 5 degree C/ambient RH

### BO_300_AMBER_CR_CLOSURE_Upright_ (5mg/mL dosage)

All the results obtained for clinical batch no: 2034827(140mL)/(5mg/mL) BO_300_AMBER_CR_CLOSURE_Upright results are well within specifications. There was no significant change in pH values, enantiomer content, degradation products and assay values of RUXOLITINIB, methyl paraben and propyl paraben for the formulation (5 mg/mL) at 5°C/ambient RH.

There is no significant change on all testing physical and chemical properties, which included assay of active and preservatives, degradation products, enantiomer, pH, and appearance up to 12 months.

### Long term condition at 25 degree C/60 percent RH

### BO_300_AMBER_CR_CLOSURE_Upright (5mg/mL dosage)

All the results obtained for clinical batch no: 2034827(140mL)/(5mg/mL) BO_300_AMBER_CR_CLOSURE_Upright results are well within specifications. There was no significant change in pH values, enantiomer content, degradation products and assay values of RUXOLITINIB, methyl paraben and propyl paraben for the formulation 25°C/60% RH.

There is no significant change on all testing physical and chemical properties, which included assay of active and preservatives, degradation products, enantiomer, pH, and appearance up to 12 months.

### Testing at 30 degree C/75 percent RH

### BO_300_AMBER_CR_CLOSURE_Upright_ (5mg/mL dosage)

All the results obtained for clinical batch no: 2034827(140mL)/(5mg/mL) BO_300_AMBER_CR_CLOSURE_Upright results are well within specifications. There was no significant change in pH values, enantiomer content, degradation products and assay values of RUXOLITINIB, methyl paraben and propyl paraben for the formulation 30°C/75% RH.

There is no significant change on all testing physical and chemical properties, which included assay of active and preservatives, degradation products, enantiomer, pH, and appearance up to 12 months.

### Testing at 40 degree C/75 percent RH

### BO_300_AMBER_CR_CLOSURE_Upright (5mg/mL dosage)

All the results obtained for clinical batch no: 2034827(140mL)/(5mg/mL) BO_300_AMBER_CR_CLOSURE_ Upright results there was no significant change in pH values, enantiomer content and assay values of RUXOLITINIB, methyl paraben and propyl paraben for the formulation (5 mg/mL) at accelerated condition 40°C/75% RH.

In degradation products 6 months analysis OOS results were observed an unspecified impurity RRT at 0.33min: 0.3% and 0.7% level results were observed. During root cause investigation it was determined to be preservative related degradation. For more details refer AQWA#2338758. Also, the 536-11 degradation product was observed at 0.5% level.

All other results (physical and chemical) remained within the specification limits for these batches.

### Testing at 5 degree C/ambient RH

### BO_300_AMBER_CR_CLOSURE_ Inverted_( 5mg/mL dosage)

All the results obtained for clinical batch no: 2034827(140mL)/(5mg/mL) BO_300_AMBER_CR_CLOSURE_Inverted results are well within specifications. There was no significant change in pH values, enantiomer content, degradation products and assay values of RUXOLITINIB, methyl paraben and propyl paraben for the formulation (5 mg/mL) at 5°C/ambient RH.

There is no significant change on all testing physical and chemical properties, which included assay of active and preservatives, degradation products, enantiomer, pH, and appearance up to 12 months.

### Long term condition at 25 degree C/60 percent RH

### BO_300_AMBER_CR_CLOSURE_ Inverted (5mg/mL dosage)

All the results obtained for clinical batch no: 2034827(140mL)/(5mg/mL) BO_300_AMBER_CR_CLOSURE_Inverted results are well within specifications. There was no significant change in pH values, enantiomer content, degradation products and assay values of RUXOLITINIB, methyl paraben and propyl paraben for the formulation 25°C/60% RH.

There is no significant change on all testing physical and chemical properties, which included assay of active and preservatives, degradation products, enantiomer, pH, and appearance up to 12 months.

### Testing at 30 degree C/75 percent RH

### BO_300_AMBER_CR_CLOSURE_ Inverted (5mg/mL dosage)

All the results obtained for clinical batch no: 2034827(140mL)/(5mg/mL) BO_300_AMBER_CR_CLOSURE_Inverted results are well within specifications. There was no significant change in pH values, enantiomer content, degradation products and assay values of RUXOLITINIB, methyl paraben and propyl paraben for the formulation 30°C/75% RH.

There is no significant change on all testing physical and chemical properties, which included assay of active and preservatives, degradation products, enantiomer, pH, and appearance up to 12 months.

### Testing at 40 degree C/75 percent RH

### BO_300_AMBER_CR_CLOSURE_Inverted (5mg/mL dosage)

All the results obtained for clinical batch no: 2034827(140mL)/(5mg/mL) BO_300_AMBER_CR_CLOSURE_Inverted results are well within specifications. There was no significant change in pH values, enantiomer content and assay values of RUXOLITINIB, methyl paraben and propyl paraben for the formulation 40°C/75% RH.

For degradation products at 3 months and 6 months analysis, 536-11 results were observed at 0.2% and 0.5% level respectively. Although these do not exceed the early phase limits set in the current specification, the increase needs to be monitored.

All other results (physical and chemical) remained within the specification limits for these batches.

### Testing at 5 degree C/ambient RH

### BO_125_AMBER_CR_CLOSURE (Placebo, 1mg/mL and 5mg/mL dosage)

Only Assay of methyl paraben and Assay of Potassium sorbate were tested and all the other physical and chemical parameters were not tested for placebo samples during stability study.

The assay values of methyl paraben and API in all tested samples didn't change significantly when the samples were stored at 5°C/ambient RH. The assay value of potassium sorbate didn't change in 5 mg/mL sugar formulation samples (T112 1215), slightly decreased from 99.3% (0-time) to 93.5% (6M) in 5 mg/mL non-sugar formulation samples (T110 1215), and significantly decreased from 94.8% (0-time) to 83.3% (6 Months) in 1 mg/mL non-sugar formulation samples. In 1 mg/mL sugar formulation samples (T111 1215), the assay value of potassium sorbate are drop around and not a stability indicator. However, the assay values of potassium sorbate decreased significantly. At 6M time point, the contents of potassium sorbate became 25% in 1 mg/mL non-sugar placebo and 48% in 5 mg/mL sugar placebo. As comparison, the decreasing of potassium sorbate was slower in sugar-formulation than that in non-sugar formulation. Three degradation products with RRT 0.42, 0.43, and 0.48 were observed at 0.1% level in 1 mg/mL non-sugar sample at 1M but only the peak with RRT 0.42 was observed at 0.1% through stability time points. A degradation peak at RRT 0.59 was observed in 1 mg/mL sugar formulation at 0.1% level at 1 month and increased to 0.2% at 3 month. No degradation peak above 0.1% in 5 mg/mL both sugar and non-sugar samples.

There was no change in the appearance and pH in all the samples up to 6months.

All results (physical and chemical) remained within the specification limits for these batches.

### BO_300BR (1mg/mL and 5mg/mL dosage)

There is no significant change on all testing physical and chemical properties, which included assay of active and preservatives, impurity profile, enantiomer, pH, and appearance, except assay value of potassium sorbate of 1 mg/mL sugar formulation at 6 M. The content of potassium sorbate in 1 mg/mL sugar formulation didn't change up to 3 M but it sharply dropped to 79.5% at 6M. No degradation product was observed above reporting threshold (0.1%).

All results (physical and chemical) remained within the specification limits for these batches.

### Accelerated testing (25 degree C/60 percent RH)

### BO_125_AMBER_CR_CLOSURE (Placebo, 1mg/mL and 5mg/mL dosage)

For placebo samples, only Assay of methyl paraben and Assay of Potassium sorbate were tested and all the other physical and chemical parameters were not tested during stability study.

No significant changes were observed on pH values, enantiomer content and the assay values of RUXOLITINIB and methyl paraben. Potassium sorbate decreased significantly during storage especially in placebo. At 6M time point, the contents of potassium sorbate became 12% in 1 mg/mL non-sugar placebo and 24% in 5 mg/mL sugar placebo. Potassium sorbate also decreased in all sample batches but not as fast as that in placebo. The assay of potassium sorbate specification is 'for information only'.

An impurity at RRT 0.43 at 0.1% level was only observed in T109 1215 (1mg/mL no sucrose formulation) 3month sample. An impurity at RRT 0.48 was observed in the same batch after1month sample, but not in 3months and 6months samples. An impurity at 0.2% level with RRT 0.59 was observed in T111 1215 (1 mg/mL sucrose formulation) 1month sample, not in 3months sample, but at 0.3% level in 6 month sample. No degradation product was observed above 0.1% in both 5 mg/mL formulations. T1091 1215 and T111 1215 appearance remained clear and colorless up to 3months, but at 6months clarity is clear and color is matching with 'Y5' (Light yellow) for batch number T109 1215 and clarity is matching with Opalescence standard IV(As per Ph.Eur) and color remains colorless for batch number T111 1215. The color of T110 1215 (5 mg/mL non-sucrose formulation) match with 'Y4'(Yellow) at 3 and 6months and clarity remains clear upto 6months. The color of T112 1215 (5 mg/mL sucrose formulation) matches with 'Y5'( Light yellow) at 3 and 6 months and clarity remains clear upto 6months. However, no degradation product was detected above reporting threshold (0.1%) in these samples.

All results (physical and chemical) remained within the specification limits for these batches.

### BO_300BR (1mg/mL and 5mg/mL dosage)

All the results obtained in BO_300BR are very similar or slightly better than those in BO_125_AMBER_CR_CLOSURE. There was no significant changes on pH values, enantiomer content and the assay values of RUXOLITINIB and methyl paraben, and assay value of potassium sorbate of two non-sugar formulations (both 1 mg/mL and 5 mg/mL). Potassium sorbate decreased significantly during storage in sugar formulation batches. However, the assay of potassium sorbate specification is 'for information only'.

T109 1215, T111 1215, and T110 1215 appearance remained colorless up to 3months, but at 6months the color change with 'Y5' (light yellow) for batch number T111 1215 and clarity remains clear for all three batches upto 6months. For batch number T112 1215 the clarity remains clear upto 6months, but the color is colorless upto 1month and at 3 months color matches with 'Y5' (light yellow) , at 6months color matches with 'Y4' (Yellow). No degradation product was observed above RT (0.1%). All results remained within the specification limits.

### Testing at 40 degree C/75 percent RH

### BO_125_AMBER_CR_CLOSURE (Placebo, 1mg/mL and 5mg/mL dosage)

For placebo samples, only Assay of methyl paraben and Assay of Potassium sorbate were tested and all the other physical and chemical parameters were not tested during stability study. Except color change and degradation profile, all the other physical and chemical testing results of the samples stored at 40°C/75%RH were very similar to those of the samples stored at 25°C/60%RH. As expected, the decreasing of potassium sorbate in the samples stored at 40°C/75%RH was faster than that at 25°C/60%RH. However, the assay of potassium sorbate specification is 'for information only'.

The degradation profiles of most samples stored at 40°C/75%RH were the same as those at 25°C/60%RH. The only difference is that the impurities 536-11 and 537-11 were detected in T110 1215 1month sample. The color of T110 1215 and T112 1215 became yellowish at 1month. No color changes were observed in T109 1215 and T111 1215 up to 3months and yellow color change was observed at 6months.

All results (physical and chemical) remained within the specification limits for these batches.

### BO_300BR (1mg/mL and 5mg/mL dosage)

Except color change, all the other physical and chemical testing results of the samples stored at 40°C/75%RH were very similar to those of the samples stored at 25°C/60%RH. The color of T111 1215 became yellowish at 3 and 6months. All results remained within the specification limits.

### Clinical batches

### In-use study

### BO_300_AMBER_CR_CLOSURE (5mg/mL dosage)

This test was performed with one clinical batch: 2034827(140mL) packaged in BO_300_AMBER_CR_CLOSURE container. Samples were stored at 25°C/60% RH up to 42 days in open bottles and analyzed.

There is no significant change on all testing physical and chemical properties, which included assay of active and preservatives, degradation products, enantiomer, pH, and appearance at 42 days.

### Freeze and thaw cycle test (20 degree C/ambient RH / 25 degree C/60 percent RH 28 days) (5mg/mL dosage)

### BO_300_AMBER_CR_CLOSURE (5mg/mL dosage)

This test was performed with one clinical batch: 2034827(140mL) packaged in BO_300_AMBER_CR_CLOSURE container. Samples were cycled at 20°C/ambient RH / 25°C/60% RH up to 28 days and analyzed.

There is no significant change on all testing physical and chemical properties, which included assay of active and preservatives, degradation products, enantiomer, pH, and appearance at 28 days.

### E. Conclusions

RUXOLITINIB solution formulation (891147, 891148) display an overall good stability behavior for 5mg/mL for the technical batch: H0004 (60mL) and H0004 (140mL) when packaged in BO_125_AMBER_CR_CLOSURE and BO_300_AMBER_CR_CLOSURE respectively and stored up to 24 months at long term condition of 25°C/60%RH, 3 months at accelerated condition of 40°C/75%RH and 24 months at 5°C/RH . All the tested parameters remained well within the proposed specifications at all tested conditions.

At 40°C/75%RH condition 6 months for both batches H0004 (60mL) and H0004 (140mL) OOS results were observed for degradation products. In batch no: H0004 (60mL) and H0004 (140mL) an unspecified impurity RRT 0.33 at 0.6% and 0.7% level results were observed respectively. During root cause investigation it was determined to be preservative related degradation. For more details refer AQWA#2184230. Also, the 536-11 degradation product was observed at 0.4% level and needs to be monitored.

RUXOLITINIB solution formulation (891147) display an overall good stability behavior for 5mg/mL for the clinical batch no: 2034827(140mL)_5mg/mL when packaged in BO_300_AMBER_CR_CLOSURE_Upright (Orientation) stored up to 12 months at long term condition of 25°C/60%RH, 3 months at accelerated condition of 40°C/75%RH, 12 months at 30°C/75%RH and 12 months at 5°C/RH . All the tested parameters remained well within the proposed specifications at all tested conditions.

At 40°C/75%RH condition 6 months for the batch: 2034827 (140mL)_Upright (Orientation), OOS results were observed for degradation products. In batch no: 2034827 (140mL)_Upright an unspecified impurity RRT 0.33 at 0.7% results were observed. During root cause investigation it was determined to be preservative related degradation. For more details refer AQWA#2338758. Also, the 536-11 degradation product was observed at 0.5% level and needs to be monitored.

RUXOLITINIB solution formulation (891147) display an overall good stability behavior for 5mg/mL for the clinical batch no: 2034827(140mL)_5mg/mL when packaged in BO_300_AMBER_CR_CLOSURE_Inverted stored up to 12 months at long term condition of 25°C/60%RH, 12 months at 30°C/75%RH and 12 months at 5°C/RH. All the tested parameters remained well within the proposed specifications at all tested conditions.

At 40°C/75%RH condition at 3 months and 6 months analysis for batch: 2034827 (140mL)_Inverted, 536-11 degradation product results were observed at 0.2% and 0.5% level respectively. Although these do not exceed the early phase limits set in the current specification, the increase needs to be monitored.

RUXOLITINIB solution formulation (891147, 891148) technical batch:H0004 (60mL) and clinical batch 2034827(140mL) were found to be chemically and physically stable for up to 42 days which support an in-use period of 42 days when stored in BO_125_AMBER_CR_CLOSURE and BO_300_AMBER_CR_CLOSURE containers respectively.

Based on the available stability data 25°C/60%RH at 24 months and 40°C/75%RH at 3 months for 5mg/mL for the technical batch:H0004 (60mL) and H0004(140mL) variants 002, the drug product storage is proposed as 'Do not store above 25°C', shelf life is proposed as '24' months. Moreover, Clinical batch: 2034827 _Upright (140mL) stability data 25°C/60%RH at 12 months, 30°C/75%RH at 12 months and 40°C/75%RH at 3 months data as well as the clinical batch 2034827 _Inverted (140mL) stability data 25°C/60%RH at 12 months, 30°C/75%RH at 12 months and 40°C/75%RH at 3 months data also supports proposed shelf life of "24'months. Based on the stability data at 40°C/75%RH, 3 month (variants 002), the transport category is assigned 'TEMPCONTROL'.

Except appearance and assay value of potassium sorbate, all the other chemical and physical testing results obtained from technical batches (variants 001) demonstrate that RUXOLITINIB 1mg/mL and 5mg/mL solution formulation are stable when stored at 40°C/75% RH (accelerated condition), 25°C/60% RH (long-term storage condition), and 5°C/ambient RH (refrigeration condition) up to 6months when packaged in BO_125_AMBER_CR_CLOSURE or BO_300BR. The appearance of tested samples was unchanged up to 6 month at 5°C/ambient RH (refrigeration condition). The color change was observed when storage at 25°C/60% RH and 40°C/75%RH. It was noticed that for the same formulation and the sample dosage strength, the samples packed in BO_300BR have better results than those in BO_125_AMBER_CR_CLOSURE. The color change of sugar formulation was faster than non-sugar formulation and the 5 mg/mL dosage strength was faster than 1 mg/mL dosage strength. All the testing physical and chemical properties which included enantiomer, assay of active ingredient, degradation products were remained within the specification limits up to 6 months.

**Table 33. Justification of shelf life, storage conditions, transport category and in-use period**

| **Dosage strength(s)** | **1mg/mL (Clinical supplies)** |
|---|---|
| **Product code(s)** | 7010282.001 and 7010281.001 |
| **Justification for shelf life and storage condition** | 21 months(7010282.001) and 18 months (7010281.001) shelf life at storage conditions 'Store at 5°C, DO NOT FREEZE' is justified. |
| | Shelf life is justified based on 6 months available data for long term storage conditions i.e 5°C/ambient RH and accelerated storage condition 25°C/60%RH for 1mg/mL technical batches. |
| **Justification for transport category** | FRIGO, DO NOT FREEZE |
| | The transport category is assigned based on 6 months available data for long term storage conditions i.e 5°C/ambient RH for 1mg/mL technical batches. |

| **Dosage strength(s)** | **5mg/mL (Clinical supplies)** |
|---|---|
| **Product code(s)** | 7010279.002 (891148), 7010280.002 (891147) |
| **Justification for shelf life and storage condition** | 24 months shelf life at storage conditions. Do not store above 25°C. |
| | Shelf life is justified based on 24 months available data for long term storage conditions i.e 25°C/60%RH and 3 months accelerated storage condition 40°C/75%RH for 5mg/mL technical batches. |
| **Justification for transport category** | TEMPCONTROL |

| **Dosage strength(s)** | **1mg/mL (Clinical supplies)** |
|---|---|
| | The transport category is assigned based on 24 months available data for long term storage conditions i.e 25°C/60%RH and 3 months accelerated storage condition 40°C/75%RH for 5mg/mL technical batches |
| **Justification of in-use period** | 42 days |
| | The compliant open bottle data at 25°C/60% RH supports the in-use period of 42 days do not store above 25°C for 5mg/mL. |
| **Product code(s)** | 7010279.001,7010280.001 |
| **Justification for shelf life and storage condition** | To be determined |

| **Dosage strength** | **1mg/mL (Bulk)** |
|---|---|
| **Product code(s)** | 7010282.001, 7010281.001 |
| **Justification for shelf life and storage condition** | 21 months(7010282.001) and 18 months(7010281.001) shelf life at storage conditions 'Store at 5°C' is justified. |
| | Shelf life is justified based on 6 months available data for long term storage conditions i.e 5°C/ambient RH and accelerated storage condition 25°C/60%RH for 1mg/mL technical batches. |
| **Justification for transport category** | FRIGO |
| | The transport category is assigned based on 6 months available data for long term storage conditions i.e 5°C/ambient RH for 1mg/mL and 5mg/mL technical batches. |
| **Dosage strength** | **5mg/mL (Bulk)** |
| **Product code(s)** | 7010279.002 (891148), 7010280.002 (891147) |
| **Justification for shelf life and storage condition** | 24 months shelf life at storage conditions. Do not store above 25°C. |

| **Dosage strength(s)** | **1mg/mL (Clinical supplies)** |
|---|---|
| | Shelf life is justified based on 24 months available data for long term storage conditions i.e 25°C/60%RH and 3 months accelerated storage condition 40°C/75%RH for 5mg/mL technical batches. |
| **Justification for transport category** | TEMPCONTROL |
| | The transport category is assigned based on 24 months available data for long term storage conditions 25°C/60%RH and 3 months accelerated storage condition 40°C/75%RH for 5mg/mL technical batches. |
| **Justification of in-use period** | 42 days |
| | The compliant open bottle data at 25°C/60% RH supports the in-use period of 42 days do not store above 25°C for 5mg/mL. |
| **Product code(s)** | 7010279.001,7010280.001 |
| **Justification for shelf life and storage condition** | To be determined |
| **Justification of in-use period** | To be determined |

### F. Technical Stability results

### Testing at 5 degree C/ambient RH, 25 degree C/60 percent RH and 40 degree C/75 percent RH Chemical and physical data: H0004 (60mL)

**Table 34. Chemical data: 5mg/mL, H0004 (60mL), BO_125 AMBER_CR_CLOSURE**

| **Storage conditions** | | **Assay** | | | | **Degradation products** | | | | | |
|---|---|---|---|---|---|---|---|---|---|---|---|
| | | Active ingredient [%] | Methyl paraben [%] | Propyl paraben [%] | RRT at 0.33 [%] | RRT at 0.51 (536-11) [%] | RRT at 0.54 [%] | RRT at 0.96 (537-11) [%] | Each Unspeci fied [%] | Enantio mer [%] | Total degradation products (Specified+ Unspecified+ Enantiomer) [%] |
| Requirements | | 90.0-110.0 | 90.0-110.0 | 90.0-110.0 | ≤ 0.5 | ≤ 0.5 | ≤ 0.5 | ≤ 0.5 | ≤ 0.5 | ≤ 0.7 | ≤ 2.0 |
| Initial analysis | | 102.4 | 101.4 | 100.4 | <RT | <RT | <RT | <RT | <RT | 0.2 | 0.2 |
| 5°C/ambient RH | 45 days | 102.4 | 100.8 | 101.7 | <RT | <RT | <RT | <RT | <RT | 0.2 | 0.2 |
| | 3 months | 101.7 | 100.3 | 100.4 | <RT | <RT | <RT | <RT | <RT | 0.2 | 0.2 |
| | 6 months | 101.0 | 97.9 | 97.5 | <RT | <RT | <RT | <RT | <RT | 0.2 | 0.2 |
| | 9 months | 103.1 | 101.8 | 101.6 | <RT | <RT | <RT | <RT | <RT | 0.2 | 0.2 |
| | 12 months | 102.4 | 98.7 | 98.5 | <RT | <RT | <RT | <RT | <RT | 0.2 | 0.2 |
| Requirements | | 90.0-110.0 | 90.0-110.0 | 90.0-110.0 | ≤ 0.5 | ≤ 0.5 | ≤ 0.5 | ≤ 0.5 | ≤ 0.5 | ≤ 0.7 | ≤ 2.0 |
| | 18 months | 101.1 | 100.1 | 99.9 | <RT | <RT | <RT | <RT | <RT | 0.2 | 0.2 |
| | 24 months | 101.0 | 99.7 | 99.4 | <RT | <RT | <RT | <RT | <RT | 0.2 | 0.2 |
| 25°C/60% RH | 45 days | 102.2 | 99.7 | 100.6 | <RT | <RT | <RT | <RT | <RT | 0.2 | 0.2 |
| | 3 months | 101.5 | 99.2 | 99.4 | <RT | <RT | <RT | <RT | <RT | 0.2 | 0.2 |
| | 6 months | 100.3 | 98.5 | 98.4 | 0.2 | <RT | <RT | <RT | 0.2 | 0.2 | 0.4 |
| | 9 months | 102.5 | 100.3 | 100.1 | 0.2 | <RT | <RT | <RT | 0.2 | 0.3 | 0.4 |
| | 12 months | 102.1 | 97.9 | 97.9 | 0.3 | <RT | <RT | <RT | 0.3 | 0.2 | 0.5 |
| | 18 months | 100.7 | 97.1 | 97.1 | 0.2 | <RT | <RT | <RT | 0.2 | 0.2 | 0.4 |
| | 24 months | 100.1 | 97.2 | 96.7 | 0.4 | <RT | <RT | <RT | 0.4 | 0.2 | 0.6 |
| 40°C/75% RH | 45 days | 101.2 | 97.8 | 98.8 | <RT | 0.2 | <RT | <RT | <RT | 0.3 | 0.4 |
| | 3 months | 101.1 | 97.3 | 97.9 | 0.3 | 0.1 | <RT | <RT | 0.3 | 0.2 | 0.7 |
| Requirements | | 90.0-110.0 | 90.0-110.0 | 90.0-110.0 | ≤ 0.5 | ≤ 0.5 | ≤ 0.5 | ≤ 0.5 ≤ | ≤ 0.5 | ≤ 0.7 | ≤ 2.0 |
| | 6 months | 97.3 | 94.8 | 94.6 | 0.6¹ | 0.4 | 0.2 | 0.1 | 0.6¹ | 0.2 | 1.6 |

| | | | | | | | | | | | |
|---|---|---|---|---|---|---|---|---|---|---|---|
| ¹OOS results observed for details refer to AQWA#2184230 | | | | | | | | | | | |

**Table 35. Physical data: 5mg/mL, H0004 (60mL), BO_125 AMBER_CR_CLOSURE**

| **Storage conditions** | | Appearance | **pH value** |
|---|---|---|---|
| Requirements | | Clear to opalescent, colorless to light yellow solution. | **For information only** |
| Initial analysis | | Clear, colorless solution | 2.7 |
| 5°C/ambient RH | 45 days | Clear, colorless solution | 2.6 |
| | 3 months | Clear, colorless solution | 2.6 |
| | 6 months | Clear, colorless solution | 2.7 |
| | 9 months | Clear, colorless solution | 2.7 |
| | 12 months | Clear, colorless solution | 2.7 |
| | 18 months | Clear, colorless solution | 2.6 |
| | 24 months | Clear, colorless solution | 2.7 |
| 25°C/60% RH | 45 days | Clear, colorless solution | 2.6 |
| | 3 months | Clear, colorless solution | 2.6 |
| | 6 months | Clear, colorless solution | 2.7 |
| | 9 months | Clear, colorless solution | 2.7 |
| | 12 months | Clear, colorless solution | 2.7 |
| | 18 months | Clear, colorless solution | 2.6 |
| | 24 months | Clear, colorless solution | 2.7 |
| 40°C/75% RH | 45 days | Clear, colorless solution | 2.6 |
| Initial analysis | | Clear, colorless solution | 2.7 |
| | 3 months | Clear, colorless solution | 2.7 |
| | 6 months | Clear, colorless solution | 2.6 |

**Table 36. Chemical data: 5mg/mL, H0004 (140mL), BO_300_AMBER_CR_CLOSURE**

| **Storage conditions** | | **Assay** | | | **Degradation products** | | | | | | |
|---|---|---|---|---|---|---|---|---|---|---|---|
| | | active ingredient [%] | Methyl paraben [%] | Propyl parabe n [%] | RRT at 0.33 [%] | RRT at 0.51 (536-11) [%] | RRT at 0.54 [%] | RRT at 0.96 537-11 [%] | Each unspecifie d [%] | Enanti omer [%] | Total degradation products(Specifi ed+Unspecified + Enantiomer) [%] |
| Requirements | | 90.0-110.0 | 90.0-110.0 | 90.0-110.0 | ≤ 0.5 | ≤ 0.5 | ≤ 0.5 | ≤ 0.5 | ≤ 0.5 | ≤ 0.7 | ≤ 2.0 |
| Initial analysis | | 103.5 | 101.4 | 100.5 | <RT | <RT | <RT | <RT | <RT | 0.2 | 0.2 |
| 5°C/ambient | RH 45 days | 102.4 | 100.6 | 101.7 | <RT | <RT | <RT | <RT | <RT | 0.2 | 0.2 |
| | 3 months | 101.4 | 100.0 | 99.9 | <RT | <RT | <RT | <RT | <RT | 0.2 | 0.2 |
| | 6 months | 101.2 | 98.9 | 98.6 | <RT | <RT | <RT | <RT | <RT | 0.2 | 0.2 |
| | 9 months | 103.5 | 102.2 | 102.0 | <RT | <RT | <RT | <RT | <RT | 0.3 | 0.3 |
| | 12 months | 102.6 | 100.2 | 100.0 | <RT | <RT | <RT | <RT | <RT | 0.2 | 0.2 |
| | 18 months | 101.9 | 100.8 | 100.7 | <RT | <RT | <RT | <RT | <RT | 0.2 | 0.2 |
| | 24 months | 100.7 | 99.6 | 99.3 | <RT | <RT | <RT | <RT | <RT | 0.2 | 0.2 |
| 25°C/60% RH | 45 days | 101.8 | 100.2 | 101.3 | <RT | <RT | <RT | <RT | <RT | 0.2 | 0.2 |
| | 3 months | 101.2 | 99.2 | 99.4 | <RT | <RT | <RT | <RT | <RT | 0.2 | 0.2 |
| Requirements | | 90.0-110.0 | 90.0-110.0 | 90.0-110.0 | ≤ 0.5 | ≤ 0.5 | ≤ 0.5 | ≤ 0.5 | ≤ 0.5 | ≤ 0.7 | ≤ 2.0 |
| Initial analysis | | 103.5 | 101.4 | 100.5 | <RT | <RT | <RT | <RT | <RT | 0.2 | 0.2 |
| | 6 months | 100.9 | 98.9 | 98.9 | 0.2 | <RT | <RT | <RT | 0.2 | 0.2 | 0.4 |
| | 9 months | 103.4 | 101.1 | 101.1 | 0.2 | <RT | <RT | <RT | 0.2 | 0.2 | 0.4 |
| | 12 months | 101.6 | 100.3 | 100.0 | 0.3 | <RT | <RT | <RT | 0.3 | 0.2 | 0.5 |
| | 18 months | 100.2 | 98.0 | 98.0 | 0.2 | <RT | <RT | 0.1 | 0.2 | 0.2 | 0.6 |
| | 24 months | 99.9 | 96.6 | 96.5 | 0.4 | <RT | <RT | <RT | 0.4 | 0.2 | 0.6 |
| 40°C/75% RH | 45 days | 100.3 | 99.3 | 100.3 | <RT | 0.1 | <RT | <RT | <RT | 0.3 | 0.4 |
| | 3 months | 99.8 | 97.8 | 98.7 | 0.3 | 0.2 | <RT | <RT | 0.3 | 0.2 | 0.7 |
| | 6 months | 97.7 | 94.1 | 94.1 | 0.7¹ | 0.4 | 0.2 | 0.1 | 0.7¹ | 0.2 | 1.7 |

| | | | | | | | | | | | |
|---|---|---|---|---|---|---|---|---|---|---|---|
| ¹OOS results observed for details refer to AQWA#2184230. | | | | | | | | | | | |

**Table 37. Physical data: 5mg/mL, H0004 (140mL), BO_300 AMBER_CR_CLOSURE**

| **Storage conditions** | | | **Appearance** | **pH value** |
|---|---|---|---|---|
| Requirements | | | Clear to opalescent, colorless to light yellow solution. | **For information only** |
| Initial analysis | | | Clear, colorless solution | 2.7 |
| | 5°C/ambient RH | 45 days | Clear, colorless solution | 2.5 |
| | | 3 months | Clear, colorless solution | 2.6 |
| | | 6 months | Clear, colorless solution | 2.7 |
| | | 9 months | Clear, colorless solution | 2.6 |
| | | 12 months | Clear, colorless solution | 2.7 |
| | | 18 months | Clear, colorless solution | 2.6 |
| | | 24 months | Clear, colorless solution | 2.7 |
| | 25°C/60% RH | 45 days | Clear, colorless solution | 2.5 |
| | | 3 months | Clear, colorless solution | 2.6 |
| | | 6 months | Clear, colorless solution | 2.7 |
| | | 9 months | Clear, colorless solution | 2.7 |
| | | 12 months | Clear, colorless solution | 2.7 |
| | | 18 months | Clear, colorless solution | 2.6 |
| | | 24 months | Clear, colorless solution | 2.7 |
| Initial analysis | | | Clear, colorless solution | 2.7 |
| | 40°C/75% RH | 45 days | Clear, colorless solution | 2.6 |
| | | 3 months | Clear, colorless solution | 2.6 |
| | | 6 months | Clear, colorless solution | 2.7 |

### G. Special tests

### Freeze and thaw cycle test

**Table 38. Chemical data: Freeze and thaw cycle test, 5mg/mL, 2034827 (140mL), BO_300 AMBER_CR_CLOSURE**

| **Storage conditions** | **Assay** | | | | | **Degradation products** | | |
|---|---|---|---|---|---|---|---|---|
| | active ingredient [%] | Methyl paraben [%] | Propyl paraben [%] | RRT at 0.3 [%] | RRT at 0.5 [%] | Each Unspecifi ed [%] | Enantio mer [%] | Total degradation products(Specifie d +Unspecified + Enantiomer [%] |
| Requirements | 90.0-110.0 | 90.0-110.0 | 90.0-110.0 | ≤ 0.5 | ≤ 0.5 | ≤ 0.5 | ≤ 0.7 | ≤ 2.0 |
| Initial analysis | 100.4 | 98.9 | 99.5 | <RT | <RT | <RT | 0.2 | 0.2 |
| -20°C/ambient RH / 25°C/60% RH 28 days | 98.6 | 98.6 | 98.9 | <RT | <RT | <RT | 0.2 | 0.2 |

**Table 39. Physical data: Freeze and thaw cycle test, 5mg/mL, 2034827 (140mL), BO_300 AMBER_CR_CLOSURE**

| **Storage conditions** | **Appearance** | **pH value** |
|---|---|---|
| Requirements | Clear to opalescent, colorless to light yellow solution. | For information only |
| Initial analysis | Clear, colorless solution | 2.6 |
| -20°C/ambient RH / 25°C/60% RH 28 days | Clear, colorless solution | 2.6 |

### In-use study

**Table 40. Chemical data: 5mg/mL, H0004 (60mL), BO_125 AMBER_CR_CLOSURE**

| | | | | | | | |
|---|---|---|---|---|---|---|---|
| **Storage conditions** | | **Assay** | | | **Degradation products** | | |

| | | active ingredient [%] | Methyl paraben [%] | Propyl paraben [%] | Each Unspecified [%] | Enantiom er [%] | Total degradation products( Specified +Unspecified +Enantiomer [%] |
|---|---|---|---|---|---|---|---|
| Requirements | | 90.0 - 110.0 | 90.0 - 110.0 | 90.0 - 110.0 | ≤ 0.5 | ≤ 0.7 | ≤ 2.0 |
| Initial analysis | | 102.5 | 100.8 | 100.5 | <RT | 0.3 | 0.3 |
| 25°C/60% RH | 30 days | 101.7 | 99.5 | 98.8 | <RT | 0.2 | 0.2 |
| | 42 days | 101.6 | 99.3 | 98.8 | <RT | 0.2 | 0.2 |

**Table 41. Physical data: 5mg/mL, H0004 (60mL), BO_125 AMBER_ CR_CLOSURE**

| **Storage conditions** | | **Appearance** | **pH value** |
|---|---|---|---|
| Requirements | | Clear to opalescent, colorless to light yellow solution. | For information only |
| Initial analysis | | Clear, colorless solution | 2.7 |
| 25°C/60% RH | 30 days | Clear, colorless solution | 2.7 |
| | 42 days | Clear, colorless solution | 2.7 |

**Table 42. Chemical data: 5mg/mL, 2034827 (140mL), BO_300 AMBER_CR_CLOSURE**

| **Storage conditions** | | **Assay** | | | **Degradation products** | | |
|---|---|---|---|---|---|---|---|
| | | active ingredient [%] | Methyl paraben [%] | Propyl paraben [%] | Each Unspecified [%] | Enantiomer [%] | Total degradation products (Specified+ Unspecified+ Enantiomer)[%] |
| Requirements | | 90.0 - 110.0 | 90.0 - 110.0 | 90.0 - 110.0 | ≤ 0.5 | ≤ 0.7 | ≤ 2.0 |
| Initial analysis | | 100.4 | 98.9 | 99.5 | <RT | 0.2 | 0.2 |
| 25°C/60% RH | 30 days | 100.1 | 98.3 | 98.5 | <RT | 0.2 | 0.2 |
| | 42 days | 100.0 | 98.9 | 99.3 | <RT | 0.2 | 0.2 |

**Table 43. Physical data: 5mg/mL, 2034827 (140mL), BO_300_AMBER_CR_CLOSURE**

| **Storage conditions** | | **Appearance** | **pH value** |
|---|---|---|---|
| Requirements | | Clear to opalescent, colorless to light yellow solution. | For information only |
| Initial analysis | | Clear, colorless solution | 2.6 |
| 25°C/60% RH | 30 days | Clear, colorless solution | 2.7 |
| | 42 days | Clear, colorless solution | 2.7 |

### H. Clinical Stability results

### Testing at 5 degree C/ambient RH, 25 degree C/60 percent RH, 30 degree C/75 percent RH and 40 degree C/75 percent RH

### Chemical and physical data: 2034827 (140mL) _Upright

**Table 44. Chemical data: 5mg/mL, 2034827 (140mL), BO_300 AMBER CR_CLOSURE**

| **Storage conditions** | | **Assay** | | | | | | **Degradation products** | | | | |
|---|---|---|---|---|---|---|---|---|---|---|---|---|
| | | Active ingredient [%] | Methyl paraben [%] | Propyl paraben [%] | RRT at 0.33 [%] | RRT at 0.49 (536-11) [%] | RRT at 0.51 [%] | RRT at 0.85 [%] | RRT at 0.96 (537-11) [%] | Each unspe cified [%] | Enanti omer [%] | Total degradation products (Specified+ Unspecified+ Enantiomer) [%] |
| Requirements | | 90.0-110.0 | 90.0-110.0 | 90.0-110 | .0 ≤ 0.5 | ≤ 0.5 | ≤ 0.5 | ≤ 0.5 | ≤ 0.5 | ≤ 0.5 | ≤ 0.7 | ≤ 2.0 |
| Initial analysis¹ | | 100.4 | 98.9 | 99.5 | <RT | <RT | <RT | <RT | <RT | <RT | 0.2 | 0.2 |
| 5°C/ambient RH | 6Weeks | 102.5 | 96.2 | 96.5 | <RT | <RT | <RT | <RT | <RT | <RT | 0.2 | 0.2 |
| | 3 months | 99.8 | 98.5 | 98.7 | <RT | <RT | <RT | <RT | <RT | <RT | 0.2 | 0.2 |
| | 6 months | 101.0 | 98.8 | 100.2 | <RT | <RT | <RT | <RT | <RT | <RT | 0.2 | 0.2 |
| | 9 months | 99.1 | 99.1 | 99.5 | <RT | <RT | <RT | <RT | <RT | <RT | 0.2 | 0.2 |

| **Storage conditions** | | **Assay** | | | | **Degradation products** | | | | | | |
|---|---|---|---|---|---|---|---|---|---|---|---|---|
| | | Active ingredient [%] | Methyl paraben [%] | Propyl paraben [%] | RRT at 0.33 [%] | RRT at 0.49 (536-11) [%] | RRT at 0.51 [%] | RRT at 0.85 [%] | RRT at 0.96 (537-11) [%] | Each unspe cified [%] | Enanti omer [%] | Total degradation products (Specified+ Unspecified+ Enantiomer) [%] |
| Requirements | | 90.0-110.C | 0 90.0-110.0 | 90.0 -110.0 | ≤ 0.5 | ≤ 0.5 | ≤ 0.5 | ≤ 0.5 | ≤ 0.5 | ≤ 0.5 | ≤ 0.7 | ≤ 2.0 |
| | 12 months | 100.4 | 99.3 | 99.7 | <RT | <RT | <RT | <RT | <RT | <RT | 0.2 | 0.2 |
| 25°C/60% RH | 6 weeks | 100.6 | 98.6 | 98.9 | <RT | <RT | <RT | <RT | <RT | <RT | 0.2 | 0.2 |
| | 3 months | 100.3 | 97.4 | 97.8 | <RT | <RT | <RT | <RT | <RT | <RT | 0.2 | 0.2 |
| | 6 months | 100.2 | 98.1 | 99.5 | 0.1 | <RT | <RT | <RT | <RT | 0.1 | 0.2 | 0.4 |
| | 9 months | 98.4 | 97.7 | 98.5 | <RT | <RT | <RT | <RT | <RT | <RT | 0.2 | 0.2 |
| | 12 months | 99.0 | 97.5 | 97.9 | <RT | <RT | <RT | <RT | <RT | <RT | 0.2 | 0.2 |
| 30°C/75% RH | 3 months | 100.5 | 98.0 | 99.4 | 0.2 | <RT | <RT | <RT | <RT | 0.2 | 0.2 | 0.4 |
| | 6 months | 100.3 | 98.9 | 99.5 | <RT | <RT | <RT | <RT | <RT | <RT | 0.2 | 0.2 |
| | 9 months | 98.7 | 96.6 | 97.1 | <RT | <RT | <RT | <RT | <RT | <RT | 0.2 | 0.2 |
| Requirements | | 90.0-110.0 | 90.0-110.0 | 90.0 -110.0 | ≤ 0.5 | ≤ 0.5 | ≤ 0.5 | ≤ 0.5 | ≤ 0.5 | ≤ 0.5 | ≤ 0.7 | ≤ 2.0 |
| | 12 months | 98.7 | 95.1 | 95.3 | 0.5 | <RT | 0.1 | <RT | <RT | 0.5 | 0.2 | 0.8 |
| | 40°C/75% RH | 99.1 | 97.0 | 97.4 | <RT | <RT | <RT | <RT | <RT | <RT | 0.2 | 0.2 |
| | 6weeks | | | | | | | | | | | |
| | 3 months | 97.3 | 95.3 | 96.6 | <RT | 0.2 | <RT | <RT | <RT | 0.2 | 0.2 | 0.4 |
| | 6 months | 96.9 | 93.5 | 95.8 | 0.7² | 0.5 | 0.1 | 0.1 | 0.1 | 0.7² | 0.3 | 1.9 |

| | | | | | | | | | | | | |
|---|---|---|---|---|---|---|---|---|---|---|---|---|
| ¹ Initial data considered from release analysis ²OOS results observed for details refer to AQWA#2338758 | | | | | | | | | | | | |

**Table 45. Physical data: 5mg/mL, 2034827(140mL), BO_300_AMBER_CR_CLOSURE**

| **Storage conditions** | | **Appearance** | **pH value** |
|---|---|---|---|
| **Requirements** | | Clear to opalescent, colorless to light yellow solution. | **For information only** |
| Initial analysis | | Clear, colorless solution | 2.6 |
| 5°C/ambient RH | 6 Weeks | Clear, colorless solution | 2.7 |
| | 3 months | Clear, colorless solution | 2.6 |
| | 6 months | Clear, colorless solution | 2.7 |
| | 9 months | Clear, colorless solution | 2.7 |
| | 12 months | Clear, colorless solution | 2.7 |
| 25°C/60% RH | 6 Weeks | Clear, colorless solution | 2.7 |
| | 3 months | Clear, colorless solution | 2.6 |
| | 6 months | Clear, colorless solution | 2.6 |
| | 9 months | Clear, colorless solution | 2.7 |
| | 12 months | Clear, colorless solution | 2.7 |
| 30°C/75% RH | 3 months | Clear, colorless solution | 2.6 |
| | 6 months | Clear, colorless solution | 2.7 |
| | 9 months | Clear, colorless solution | 2.6 |
| | 12 months | Clear, colorless solution | 2.7 |
| 40°C/75% RH | 6 Weeks | Clear, colorless solution | 2.7 |
| **Requirements** | | Clear to opalescent, colorless to light yellow solution. | **For information only** |
| Initial analysis | | Clear, colorless solution | 2.6 |
| | 3 months | Clear, colorless solution | 2.6 |
| | 6 months | Clear, colorless solution | 2.7 |

### Chemical and physical data: 2034827 (140mL) _ Inverted

**Table 46. Chemical data: 5mg/mL, 2034827 (140mL), BO_300 AMBER_CR_CLOSURE**

| **Storage conditions** | | **Assay** | | | **Degradation products** | | | | | | |
|---|---|---|---|---|---|---|---|---|---|---|---|
| | | Active ingredient [%] | Methyl paraben [%] | Propyl paraben [%] | RRT at 0.33 [%] | RRT at 0.51 (536-11) [%] | RRT at 0.55 [%] | RRT at 0.96 (537-11) [%] | Each unspe cified [%] | Enantio mer [%] | Total degradation products (Specified+ Unspecified+ Enantiomer) [%] |
| Requirements | | 90.0-110.0 | 90.0-110.0 | 90.0 -110.0 | ≤ 0.5 | ≤ 0.5 | ≤ 0.5 | ≤ 0.5 | ≤ 0.5 | ≤ 0.7 | ≤ 2.0 |
| Initial analysis¹ | | 100.4 | 98.9 | 99.5 | <RT | <RT | <RT | <RT | <RT | 0.2 | 0.2 |
| 5°C/ambient RH | 6Weeks | 100.9 | 98.8 | 100.1 | <RT | <RT | <RT | <RT | <RT | 0.2 | 0.2 |
| | 3 months | 101.1 | 99.7 | 100.4 | <RT | <RT | <RT | <RT | <RT | 0.2 | 0.2 |
| Requirements | | 90.0-110.0 | 90.0-110.0 | 90.0 -110.0 | **≤ 0.5** | ≤ 0.5 | ≤ 0.5 | ≤ 0.5 | ≤ 0.5 | ≤ 0.7 | ≤ 2.0 |
| | 6 months | 99.9 | 99.2 | 99.2 | <RT | <RT | <RT | <RT | <RT | 0.2 | 0.2 |
| | 9 months | 99.5 | 98.6 | 99.1 | <RT | <RT | <RT | <RT | <RT | 0.2 | 0.2 |
| | 12 months | 99.5 | 98.5 | 99.0 | <RT | <RT | <RT | <RT | <RT | 0.2 | 0.2 |
| 25°C/60% RH | 3 months | 100.6 | 99.1 | 99.8 | <RT | <RT | <RT | <RT | <RT | 0.2 | 0.2 |
| | 6 months | 98.9 | 98.5 | 98.5 | <RT | <RT | <RT | <RT | <RT | 0.2 | 0.2 |
| | 9 months | 98.6 | 98.1 | 98.1 | 0.4 | <RT | <RT | <RT | 0.4 | 0.2 | 0.6 |
| | 12 months | 98.9 | 97.0 | 97.3 | 0.3 | <RT | <RT | <RT | 0.3 | 0.2 | 0.5 |
| 30°C/75% RH | 3 months | 100.5 | 99.0 | 99.7 | <RT | <RT | <RT | <RT | <RT | 0.2 | 0.2 |
| | 6 months | 98.6 | 98.2 | 98.3 | <RT | <RT | <RT | <RT | <RT | 0.2 | 0.2 |
| | 9 months | 98.8 | 96.9 | 97.3 | 0.4 | 0.1 | <RT | <RT | 0.4 | 0.2 | 0.7 |

| | | Active ingredient [%] | Methyl paraben [%] | Propyl paraben [%] | RRT at 0.33 [%] | RRT at 0.51 (536-11) [%] | RRT at 0.55 [%] | RRT at 0.96 (537-11) [%] | Each unspe cified [%] | [ Enantio mer %] | Total degradation products (Specified+ Unspecified+ Enantiomer) [%] |
|---|---|---|---|---|---|---|---|---|---|---|---|
| Requirements | | 90.0-110.0 | 90.0-110.0 | 90.0 -110.0 | ≤ 0.5 | ≤ 0.5 | ≤ 0.5 | ≤ 0.5 | ≤ 0.5 | ≤ 0.7 | ≤ 2.0 |
| | 12 months | 98.5 | 96.1 | 96.6 | 0.4 | 0.1 | <RT | <RT | 0.4 | 0.2 | 0.7 |
| 40°C/75% RH | 6 Weeks | 99.9 | 97.3 | 99.0 | 0.2 | <RT | <RT | <RT | 0.2 | 0.2 | 0.4 |
| | 3 months | 99.9 | 96.9 | 97.3 | <RT | 0.2 | <RT | <RT | 0.2 | 0.2 | 0.4 |
| | 6 months | 94.3 | 92.4 | 93.5 | 0.4 | 0.5 | 0.2 | 0.1 | 0.5 | 0.2 | 1.4 |

| | | | | | | | | | | | |
|---|---|---|---|---|---|---|---|---|---|---|---|
| ¹ Initial data considered from release analysis | | | | | | | | | | | |

**Table 47. Physical data: 5mg/mL, 2034827(140mL), BO_300_AMBER_CR_CLOSURE**

| **Storage conditions** | | **Appearance** | **pH value** |
|---|---|---|---|
| **Requirements** | | Clear to opalescent, colorless to light yellow solution. | For information only |
| Initial analysis | | Clear, colorless solution | 2.6 |
| 5°C/ambient RH | 6 Weeks | Clear, colorless solution | 2.7 |
| | 3 months | Clear, colorless solution | 2.7 |
| | 6 months | Clear, colorless solution | 2.6 |
| | 9 months | Clear, colorless solution | 2.7 |
| | 12 months | Clear, colorless solution | 2.7 |
| 25°C/60% RH | 3 months | Clear, colorless solution | 2.7 |
| | 6 months | Clear, colorless solution | 2.6 |
| | 9 months | Clear, colorless solution | 2.7 |
| | 12 months | Clear, colorless solution | 2.7 |
| 30°C/75% RH | 3 months | Clear, colorless solution | 2.7 |
| | 6 months | Clear, colorless solution | 2.7 |
| | 9 months | Clear, colorless solution | 2.7 |
| | 12 months | Clear, colorless solution | 2.7 |
| 40°C/75% RH | 6 Weeks | Clear, colorless solution | 2.7 |
| | 3 months | Clear, colorless solution | 2.7 |
| Initial analysis | | Clear, colorless solution | 2.6 |
| | 6 months | Clear solution, color matches with "Y5"(Light yellow) | 2.6 |

### Stability results

### Testing at 5 degree C/ambient RH, 25 degree C/60 percent RH and 40 degree C/75 percent RH Placebo solution

### 1. Chemical and physical data: T010 0216

**Table 48. Chemical data: 1mg/mL (Placebo solution), T010 0216, BO_125 AMBER_CR_CLOSURE**

| **Storage conditions** | | | **Assay** | | | **Degradation products** | | **Enantiome r [%]** |
|---|---|---|---|---|---|---|---|---|
| | | | **Active ingredient** [%] | **Potassium sorbate** [%] | **Methyl paraben** [%] | **RRT [%]** | **Total** [%] | |
| **Requirements** | | | **90.0 -110.0** | **For information only** | **For information only** | **≤ 0.5** | **≤ 2.0** | **≤ 0.7** |
| Initial analysis | | | n.a | 97.0 | 111.1 | n.a | n.a | n.a |
| | 5°C/ambient RH | 1 month | n.a | 108.3 | 112.9 | n.a | n.a | n.a |
| | | 3 months | n.a | 71.9¹ | 109.1 | n.a | n.a | n.a |
| | | 6 months | n.a | 24.71 | 120.5 | n.a | n.a | n.a |
| Initial analysis | | | n.a | 97.0 | 111.1 | n.a | n.a | n.a |
| | 25°C/60% RH | 1 month | n.a | 42.1¹ | 112.0 | n.a | n.a | n.a |
| | | 3 months | n.a | 85.5¹ | 112.1 | n.a | n.a | n.a |
| | | 6 months | n.a | 11.5¹ | 120.1 | n.a | n.a | n.a |
| | 40°C/75% RH | 1 month | n.a | 25.8¹ | 111.6 | n.a | n.a | n.a |
| | | 3 months | n.a | 11.9¹ | 108.8 | n.a | n.a | n.a |
| | | 6 months | n.a | 9.7¹ | 117.1 | n.a | n.a | n.a |

| | | | | | | | | |
|---|---|---|---|---|---|---|---|---|
| n.a Not Applicable ¹ For information only, there is no limit, however formulation discontinued. | | | | | | | | |

**Table 49. Physical data: 1mg/mL (Placebo solution), T010 0216, BO_125 AMBER_CR_CLOSURE**

| **Storage conditions** | | **Appearance'** | **pH value¹** |
|---|---|---|---|
| **Requirements** | | **For information only** | **For information only** |
| Initial analysis | | n.a | n.a |
| 5°C/ambient RH | 1 month | n.a | n.a |
| | 3 months | n.a | n.a |
| | 6 months | n.a | n.a |
| 25°C/60% RH | 1 month | n.a | n.a |
| | 3 months | n.a | n.a |
| | 6 months | n.a | n.a |
| 40°C/75% RH | 1 month | n.a | n.a |
| | 3 months | n.a | n.a |
| | 6 months | n.a | n.a |

| | | | |
|---|---|---|---|
| n.a Not Applicable ¹ Optional tests | | | |

**Table 50. Chemical data: 5mg/mL (Placebo solution), T011 0216, BO_125 AMBER_CR_CLOSURE**

| **Storage conditions** | | | **Assay** | | | **Degradation products** | | **Enantiomer** |
|---|---|---|---|---|---|---|---|---|
| | | | **Active ingredient** [%] | **Potassium sorbate** [%] | **Methyl paraben** [%] | **RRT** [%] | **Total** [%] | **[%]** |
| **Requirements** | | | **90.0 -110.0** | **For information only** | **For information only** | **≤ 0.5** | **≤ 2.0** | **≤ 0.7** |
| Initial analysis | | | n.a | 90.0 | 116.3 | n.a | n.a | n.a |
| | 5°C/ambient RH | 1 month | n.a | 60.3 | 114.5 | n.a | n.a | n.a |
| | | 3 months | n.a | 87.6 | 114.7 | n.a | n.a | n.a |
| | | 6 months | n.a | 48.2 | 118.5 | n.a | n.a | n.a |
| | 25°C/60% RH | 1 month | n.a | 64.9 | 114.2 | n.a | n.a | n.a |
| | | 3 months | n.a | 48.0 | 110.5 | n.a | n.a | n.a |
| | | 6 months | n.a | 23.7 | 117.3 | n.a | n.a | n.a |
| | 40°C/75% RH | 1 month | n.a | 99.2 | 114.3 | n.a | n.a | n.a |
| | | 3 months | n.a | 43.4 | 112.6 | n.a | n.a | n.a |
| | | 6 months | n.a | 17.8 | 114.2 | n.a | n.a | n.a |

| | | | | | | | | |
|---|---|---|---|---|---|---|---|---|
| n.a Not Applicable | | | | | | | | |

**Table 51. Physical data: 5mg/mL (Placebo solution), T011 0216, BO_125 AMBER_CR_CLOSURE**

| **Storage conditions** | | | **Appearance¹** | **pH value¹** |
|---|---|---|---|---|
| **Requirements** | | | **For information only** | **For information only** |
| Initial analysis | | | n.a | n.a |
| | 5°C/ambient RH | 1 month | n.a | n.a |
| | | 3 months | n.a | n.a |
| | | 6 months | n.a | n.a |
| | 25°C/60% RH | 1 month | n.a | n.a |
| | | 3 months | n.a | n.a |
| | | 6 months | n.a | n.a |
| | 40°C/75% RH | 1 month | n.a | n.a |
| | | 3 months | n.a | n.a |
| | | 6 months | n.a | n.a |

| | | | | |
|---|---|---|---|---|
| n.a Not Applicable ¹ Optional tests | | | | |

### BO_125 AMBER_CR_CLOSURE

### Chemical and physical data

**Table 12. Chemical data: 1mg/mL, T109 1215, BO_125 AMBER_CR_CLOSURE**

| **Storage conditions** | | | **Assay** | | | **Degradation products** | | | | **Enantiomer** |
|---|---|---|---|---|---|---|---|---|---|---|
| | | | **Active ingredient** [%] | **Potassium sorbate** [%] | **Methyl paraben** [%] | **0.42 RRT** [%] | **0.43 RRT** [%] | **0.48 RRT** [%] | **Total** [%] | [%] |
| **Requirements** | | | **90.0-110.0** | **For information only** | **For information only** | ≤ 0.5 | ≤ 0.5 | ≤ 0.5 | ≤ 2.0 | ≤ 0.7 |
| | | Initial analysis | 102.0 | 94.8 | 99.2 | <RT | <RT | <RT | <RT | 0.2 |
| | 5°C/ambient RH | 1 month | 101.1 | 85.7 | 99.4 | 0.1 | 0.1 | 0.1 | 0.4 | 0.2 |
| | | 3 months | 101.1 | 98.5 | 98.3 | <RT | 0.1 | <RT | 0.1 | 0.2 |
| | | 6 months | 103.0 | 83.3 | 107.7 | <RT | <RT | <RT | <RT | 0.2¹ |
| | 25°C/60% RH | 1 month | 101.6 | 92.1 | 99.9 | <RT | <RT | 0.1 | 0.1 | 0.2 |
| | | 3 months | 101.3 | 91.9 | 98.2 | <RT | 0.1 | <RT | 0.1 | 0.2 |
| | | 6 months | 101.8 | 61.2 | 107.0 | <RT | <RT | <RT | <RT | 0.2¹ |
| | 40°C/75% RH | 1 month | 101.4 | 95.6 | 99.2 | <RT | <RT | 0.1 | 0.1 | 0.2 |
| | | 3 months | 101.6 | 95.4 | 97.2 | <RT | 0.1 | <RT | 0.1 | 0.2 |
| | | 6 months | 101.8 | 61.2 | 103.8 | <RT | <RT | <RT | <RT | 0.2¹ |

| | | | | | | | | | | |
|---|---|---|---|---|---|---|---|---|---|---|
| ¹ For enantiomer test flow rate is not followed as per test method (1.0ml/minute is followed instead of 0.8ml/min ), for details refer to deviation # 1618342 | | | | | | | | | | |

**Table 53. Physical data: 1mg/mL, T109 1215, BO_125 AMBER_CR_CLOSURE**

| **Storage conditions** | | | **Appearance** | **pH value** |
|---|---|---|---|---|
| **Requirements** | | | **For information only** | **For information only** |
| Initial analysis | | | Clear, colorless solution | 2.86 |
| | 5°C/ambient RH | 1 month | Clear, colorless solution | 2.83 |
| | | 3 months | Clear, colorless solution | 2.87 |
| | | 6 months | Clear, colorless solution | 2.86 |
| | 25°C/60% RH | 1 month | Clear, colorless solution | 2.84 |
| | | 3 months | Clear, colorless solution | 2.88 |
| | | 6 months | Clear, color matches with 'Y5' (Light yellow) | 2.86 |
| | 40°C/75% RH | 1 month | Clear, colorless solution | 2.85 |
| | | 3 months | Clear, colorless solution | 2.89 |
| | | 6 months | Clear, color matches with 'Y5' (Light yellow) | 2.87 |

**Table 54. Chemical data: 1mg/mL, T111 1215, BO_125 AMBER_CR_CLOSURE**

| **Storage conditions** | | | **Assay** | | | **Degradation products** | | | | | **Enantiomer** [%] |
|---|---|---|---|---|---|---|---|---|---|---|---|
| | | | **Active ingredient** [%] | **Potassium sorbate** [%] | **Methyl paraben** [%] | **0.42 RRT** [%] | **0.43 RRT** [%] | **0.48 RRT** [%] | **0.59 RRT** [%] | **Total** [%] | |
| **Requirements** | | | **90.0 -110.0** | **For information only** | **For information only** | **≤ 0.5** | **≤ 0.5** | **≤ 0.5** | **≤ 0.5** | **≤ 2.0** | **≤ 0.7** |
| | | Initial analysis | 95.8 | 86.5 | 88.4 | <RT | <RT | <RT | <RT | <RT | 0.2 |
| | 5°C/ambient RH | 1 month | 99.3 | 95.5 | 94.2 | <RT | <RT | <RT | 0.1 | 0.1 | 0.1 |
| | | 3 months | 102.6 | 97.0 | 97.4 | <RT | <RT | <RT | <RT | <RT | 0.2 |
| | | 6 months | 101.3³ | 90.3 | 101.1 | <RT | <RT | <RT | 0.2 | 0.2 | 0.2² |
| | 25°C/60% RH | 1 month | 97.8 | 92.0 | 93.4 | <RT | <RT | <RT | 0.2 | 0.2 | 0.2¹ |
| | | 3 months | 101.6 | 63.0 | 96.2 | <RT | <RT | <RT | <RT | <RT | 0.2 |
| | | 6 months | 103.2 | 80.2 | 104.3 | <RT | <RT | <RT | 0.3 | 0.3 | 0.2² |
| | 40°C/75% RH | 1 month | 95.8 | 85.6 | 91.6 | <RT | <RT | <RT | 0.1 | 0.1 | 0.1 |
| | | 3 months | 102.7 | 80.9 | 97.0 | <RT | <RT | <RT | <RT | <RT | 0.2 |
| | | 6 months | 100.5³ | 67.3 | 101.5 | <RT | <RT | <RT | 0.1 | 0.1 | 0.1² |

| | | | | | | | | | | | |
|---|---|---|---|---|---|---|---|---|---|---|---|
| ¹ OOS is observed due to mis-labelling, for details refer to AQWA # 1548736. ² For enantiomer test flow rate is not followed as per test method (1.0ml/minute is followed instead of 0.8ml/min ), for details refer to deviation # 1618342 ³ OOS is observed due to mis-labelling, for details refer to AQWA # 1615459 and product deviation #1628150. | | | | | | | | | | | |

**Table 55. Physical data: 1mg/mL, T111 1215, BO_125 AMBER_CR_CLOSURE**

| **Storage conditions** | | **Appearance** | **pH value** |
|---|---|---|---|
| **Requirements** | | **For information only** | **For information only** |
| | Initial analysis | Clear, colorless solution | 2.89 |
| 5°C/ambient RH | 1 month | Clear, colorless solution | 2.86 |
| | 3 months | Clear, colorless solution | 2.88 |
| | 6 months | Clear, colorless solution | 2.87 |
| 25°C/60% RH | 1 month | Clear, colorless solution | 2.87 |
| | 3 months | Clear, colorless solution | 2.86 |
| | 6 months | Matches with opalescence standard IV, colorless solution | 2.91 |
| 40°C/75% RH | 1 month | Clear, colorless solution | 2.87 |
| | 3 months | Opalescence and matches with reference suspension IV, colorless solution | 2.98 |
| | 6 months | Matches with opalescence standard IV, color matches with 'Y2' (Dark yellow) | 2.93 |

**Table 56. Chemical data: 5mg/mL, T110 1215, BO_125 AMBER_CR_CLOSURE**

| **Storage conditions** | | | **Assay** | | | **Degradation products** | | | | | | **Enantiomer** |
|---|---|---|---|---|---|---|---|---|---|---|---|---|
| | | | **Active ingredient [%]** | **Potassium sorbate [%]** | **Methyl paraben [%]** | **536-11 [%]** | **537-11 [%]** | **0.42 RRT [%]** | **0.43 RRT [%]** | **0.48 RRT [%]** | **Total [%]** | **[%]** |
| **Requirements** | | | **90.0-110.0** | **For information only** | **For information only** | **≤ 0.5** | **≤ 0.5** | **≤ 0.5** | **≤ 0.5** | **≤ 0.5** | **≤ 2.0** | **≤ 0.7** |
| Initial analysis | | | 100.5 | 99.3 | 102.3 | <RT | <RT | <RT | <RT | <RT | <RT | 0.2 |
| | 5°C/ambient RH | 1 month | 101.5 | 99.8 | 104.3 | <RT | <RT | <RT | <RT | <RT | <RT | 0.1 |
| | | 3 months | 100.0 | 96.3 | 100.4 | <RT | <RT | <RT | <RT | <RT | <RT | 0.2 |
| | | 6 months | 102.0 | 93.5 | 103.8 | <RT | <RT | <RT | <RT | <RT | <RT | 0.3¹ |
| | 25°C/60% RH | 1 month | 101.3 | 87.7 | 104.5 | <RT | <RT | <RT | <RT | <RT | <RT | 0.1 |
| | | 3 months | 101.8 | 82.5 | 104.1 | <RT | <RT | <RT | <RT | <RT | <RT | 0.2 |
| | | 6 months | 101.3 | 74.7 | 103.1 | <RT | <RT | <RT | <RT | <RT | <RT | 0.2¹ |
| | 40°C/75% RH | 1 month | 100.1 | 38.8 | 105.0 | 0.2 | 0.1 | <RT | <RT | <RT | 0.3 | 0.1 |
| | | 3 months | 101.6 | 71.0 | 102.9 | <RT | <RT | <RT | <RT | <RT | <RT | 0.2 |
| | | 6 months | 102.2 | 53.2 | 103.1 | <RT | <RT | <RT | <RT | <RT | <RT | 0.2¹ |

| | | | | | | | | | | | | |
|---|---|---|---|---|---|---|---|---|---|---|---|---|
| ¹ For enantiomer test flow rate is not followed as per test method (1.0ml/minute is followed instead of 0.8ml/min ), for details refer to deviation # 1618342 | | | | | | | | | | | | |

**Table 57. Physical data: 5mg/mL, T110 1215, BO_125 AMBER_CR_CLOSURE**

| **Storage conditions** | | | **Appearance** | **pH value** |
|---|---|---|---|---|
| **Requirements** | | | **For information only** | **For information only** |
| Initial analysis | | | Clear, colorless solution | 2.84 |
| | 5°C/ambient RH | 1 month | Clear, colorless solution | 2.83 |
| | | 3 months | Clear, colorless solution | 2.86 |
| | | 6 months | Clear, colorless solution | 2.84 |
| | 25°C/60% RH | 1 month | Clear, colorless solution | 2.83 |
| | | 3 months | Clear, color matches with 'Y4' (Yellow) | 2.86 |
| | | 6 months | Clear, color matches with 'Y4' (Yellow) | 2.84 |
| | 40°C/75% RH | 1 month | Clear, color matches with 'Y3' (Yellow) | 2.81 |
| | | 3 months | Clear, color matches with 'Y4' (Yellow) | 2.87 |
| | | 6 months | Clear, color matches with 'Y3' (Yellow | 2.85 |

**Table 58. Chemical data: 5mg/mL, T112 1215, BO_125 AMBER_CR_CLOSURE**

| **Storage conditions** | | | **Assay** | | | | **Degradation products** | | | | | | **Enantiomer** |
|---|---|---|---|---|---|---|---|---|---|---|---|---|---|
| | | | **Active ingredient** [%] | **Potassium** sorbate [%] | **Methyl paraben** [%] | **536-11** [%] | **537-11** [%] | **519-11** [%] | **0.42 RRT** [%] | **0.43 RRT** [%] | **0.48 RRT** [%] | **Total** [%] | [%] |
| **Requirements** | | | **90.0 - 110.0** | **For** information only | **For information** only | **≤ 0.5** | **≤ 0.5** | **≤ 0.5** | **≤ 0.5** | **≤ 0.5** | **≤ 0.5** | **≤ 2.0** | **≤ 0.7** |
| Initial analysis | | | 101.2 | 101.2 | 102.9 | <RT | <RT | <RT | <RT | <RT | <RT | <RT | 0.3 |
| | 5°C/ambient RH | 1 month | 100.8 | 100.1 | 101.8 | <RT | <RT | <RT | <RT | <RT | <RT | <RT | 0.2 |
| | | 3 months | 102.1 | 101.4 | 103.1 | <RT | <RT | <RT | <RT | <RT | <RT | <RT | 0.3 |
| | | 6 months | 102.8 | 101.2 | 103.9 | <RT | <RT | <RT | <RT | <RT | <RT | <RT³ | 0.3² |
| | 25°C/60% RH | 1 month | 100.8 | 96.9 | 102.0 | <RT | <RT | <RT | <RT | <RT | <RT | <RT | 0.2 |
| | | 3 months | 101.5¹ | 92.0¹ | 100.9¹ | <RT | <RT | <RT | <RT | <RT | <RT | <RT | 0.3 |
| | | 6 months | 102.7 | 83.7 | 103.9 | <RT | <RT | <RT | <RT | <RT | <RT | <RT³ | 0.3² |
| | 40°C/75% RH | 1 month | 101.1 | 85.8 | 102.5 | <RT | <RT | <RT | <RT | <RT | <RT | <RT | 0.2 |
| | | 3 months | 101.6 | 71.5 | 101.7 | <RT | <RT | <RT | <RT | <RT | <RT | <RT | 0.3 |
| | | 6 months | 101.8 | 61.1 | 101.8 | <RT | <RT | 0.1 | <RT | <RT | <RT | 0.1 | 0.3² |

| **Storage conditions** | **Assay** | | | | **Degradation products** | | | | | | **Enantiomer** | | |
|---|---|---|---|---|---|---|---|---|---|---|---|---|---|
| | **Active ingredient** [%] | **Potassium sorbate** [%] | **Methyl paraben** [%] | **536-11** [%] | **537-11** [%] | **519-11** [%] | **0.42 RRT** [%] | 0.43 RRT [%] | **0.48 RRT** [%] | **Total** [%] | [%] | | |
| **Requirements** | **90.0** - **110.0** | **For information only** | **For information only** | **≤ 0.5** | **≤ 0.5** | **≤ 0.5** | **≤ 0.5** | **≤ 0.5** | **≤ 0.5** | **≤ 2.0** | **≤ 0.7** | | |
| Initial analysis | 101.2 | 101.2 | 102.9 | <RT | <RT | <RT | <RT | <RT | <RT | <RT | 0.3 | | |

| | | | | | | | | | | | | | |
|---|---|---|---|---|---|---|---|---|---|---|---|---|---|
| ¹ Reanalysis results are reports, for for details refer A QWA # 15678 35 ² For enantiomer test flow rate is not followed as per test method (1.0ml/minute is followed instead of 0.8ml/min ), for details refer to deviation # 1618342. ³ OOE is observed, for details refer AQWA # 1617159. | | | | | | | | | | | | | |

**Table 59. Physical data: 5mg/mL, T112 1215, BO_125 AMBER_CR_CLOSURE**

| **Storage conditions** | | | **Appearance** | **pH value** |
|---|---|---|---|---|
| **Requirements** | | | **For information only** | **For information only** |
| Initial analysis | | | Clear, colorless solution | 2.82 |
| | 5°C/ambient RH | 1 month | Clear, colorless solution | 2.80 |
| | | 3 months | Clear, colorless solution | 2.85 |
| | | 6 months | Clear, colorless solution | 2.82 |
| | 25°C/60% RH | 1 month | Clear, colorless solution | 2.80 |
| | | 3 months | Clear, color matches with 'Y5' (light yellow) | 2.85 |
| | | 6 months | Clear, color matches with 'Y5' (light yellow) | 2.82 |
| | 40°C/75% RH | 1 month | Clear, color matches with 'Y6' (light yellow) | 2.81 |
| | | 3 months | Clear, color matches with 'Y3' (Yellow) | 2.87 |
| | | 6 months | Clear, color matches with 'Y2' (dark yellow) | 2.84 |

### BO_300BR

### Chemical and physical data

**Table 60. Chemical data: 1mg/mL, T109 1215, BO_300BR**

| **Storage conditions** | | | **Assay** | | | **Degradation products** | | | | | | | **Enantiomer** |
|---|---|---|---|---|---|---|---|---|---|---|---|---|---|
| | | | **Active ingredient** [%] | **Potassium sorbate [%]** | **Methyl paraben [%]** | **536-**11 [%] | **537-**11 [%] | **0.42 RRT** [%] | **0.43 RRT** [%] | **0.48 RRT** [%] | **0.66** RRT [%] | **Total** [%] | [%] |
| **Requirements** | | | **90.0 -110.0** | **For informatio n only** | **For information only** | **≤ 0.5** | **≤ 0.5** | **≤ 0.5** | **≤ 0.5** | **≤ 0.5** | **≤ 0.5** | **≤ 2.0** | **≤ 0.7** |
| | | Initial analysis | 104.4 | 93.0 | 99.7 | <RT | <RT | <RT | <RT | <RT | <RT | <RT | 0.2 |
| | 5°C/ambient RH | 1 month | 105.2 | 96.7 | 100.9 | <RT | <RT | <RT | <RT | <RT | <RT | <RT | 0.2 |
| | | 3 months | 104.7 | 90.0 | 100.0 | <RT | <RT | <RT | <RT | <RT | <RT | <RT | 0.2 |
| | | 6 months | 107.1 | 93.6 | 108.5 | <RT | <RT | <RT | <RT | <RT | <RT | <RT | 0.2¹ |
| | 25°C/60% RH | 1 month | 104.8 | 90.4 | 100.3 | <RT | <RT | <RT | <RT | <RT | <RT | <RT | 0.1 |
| | | 3 months | 104.5 | 92.3 | 99.3 | <RT | <RT | <RT | <RT | <RT | <RT | <RT | 0.2 |
| | | 6 months | 100.0 | 106.1 | 108.5 | <RT | <RT | <RT | <RT | <RT | <RT | <RT | 0.3¹ |
| | 40°C/75% RH | 1 month | 105.8 | 96.3 | 100.5 | <RT | <RT | <RT | <RT | <RT | <RT | <RT | 0.2 |
| | | 3 months | 105.0 | 90.2 | 98.8 | <RT | <RT | <RT | <RT | <RT | <RT | <RT | 0.2 |
| | | 6 months | 106.7 | 90.3 | 119.5 | <RT | <RT | <RT | <RT | <RT | 0.1 | 0.1 | 0.2¹ |

| | | | | | | | | | | | | | |
|---|---|---|---|---|---|---|---|---|---|---|---|---|---|
| ¹ For enantiomer test flow rate is not followed as per test method (1.0ml/minute is followed instead of 0.8ml/min ), for details refer to deviation # 1618342. | | | | | | | | | | | | | |

**Table 61. Physical data: 1mg/mL, T109 1215, BO_300BR**

| **Storage conditions** | | | **Appearance** | **pH value** |
|---|---|---|---|---|
| Requirements | | | **For information only** | **For information only** |
| | | Initial analysis | Clear, colorless solution | 2.87 |
| | 5°C/ambient RH | 1 month | Clear, colorless solution | 2.84 |
| | | 3 months | Clear, colorless solution | 2.87 |
| | | 6 months | Clear, colorless solution | 2.86 |
| | 25°C/60% RH | 1 month | Clear, colorless solution | 2.84 |
| | | 3 months | Clear, colorless solution | 2.87 |
| | | 6 months | Clear, colorless solution | 2.92 |
| | 40°C/75% RH | 1 month | Clear, colorless solution | 2.86 |
| | | 3 months | Clear, colorless solution | 2.90 |
| | | 6 months | Clear, colorless solution | 2.89 |

**Table 62. Chemical data: 1mg/mL, T111 1215, BO_300BR**

| **Storage conditions** | | **Assay** | | | **Degradation products** | | | | | | | **Enantiome** |
|---|---|---|---|---|---|---|---|---|---|---|---|---|
| | | **Active ingredient [%]** | **Potassium sorbate [%]** | **Methyl paraben [%]** | **536-11** [%] | **537-11** [%] | **0.12** RRT [%] | **0.42 RRT** [%] | **0.43 RRT** [%] | **0.48 RRT** [%] | **Total** [%] | r **[%]** |
| **Requirements** | | **90.0 - 110.0** | **For informatio n only** | **For informatio n only** | **≤ 0.5** | **≤ 0.5** | **≤ 0.5** | **≤ 0.5** | **≤ 0.5** | **≤ 0.5** | **≤ 2.0** | **≤ 0.7** |
| | Initial analysis | 102.0 | 91.5 | 96.1 | <RT | <RT | <RT | <RT | <RT | <RT | <RT | 0.2 |
| 5°C/ambient RH | 1 month | 99.9 | 96.2 | 94.9 | <RT | <RT | <RT | <RT | <RT | <RT | <RT | 0.1 |
| | 3 months | 102.2 | 92.8 | 96.5 | <RT | <RT | <RT | <RT | <RT | <RT | <RT | 0.2 |
| | 6 months | 102.5 | 79.5 | 103.1 | <RT | <RT | <RT | <RT | <RT | <RT | <RT | 0.2¹ |
| 25°C/60% RH | 1 month | 101.2 | 81.9 | 96.5 | <RT | <RT | <RT | <RT | <RT | <RT | <RT | 0.1 |
| | 3 months | 102.5 | 75.7 | 96.8 | <RT | <RT | <RT | <RT | <RT | <RT | <RT | 0.2 |
| | 6 months | 101.9 | 47.6 | 103.9 | <RT | <RT | <RT | <RT | <RT | <RT | <RT | 0.2¹ |
| 40°C/75% RH | 1 month | 98.4 | 80.9 | 93.9 | <RT | <RT | <RT | <RT | <RT | <RT | <RT | 0.1 |
| | 3 months | 101.2 | 58.3 | 95.3 | <RT | <RT | 0.1 | <RT | <RT | <RT | 0.1 | 0.2 |
| | 6 months | 101.3 | 42.7 | 100.9 | 0.1 | <RT | <RT | <RT | <RT | <RT | 0.1 | 0.2¹ |

| | | | | | | | | | | | | |
|---|---|---|---|---|---|---|---|---|---|---|---|---|
| ¹ For enantiomer test flow rate is not followed as per test method (1.0ml/minute is followed instead of 0.8ml/min ), for details refer to deviation # 1618342. | | | | | | | | | | | | |

**Table 63. Physical data: 1mg/mL, T111 1215, BO_300BR**

| **Storage conditions** | | | **Appearance** | **pH value** |
|---|---|---|---|---|
| **Requirements** | | | **For information only** | **For information only** |
| | | Initial analysis | Clear, colorless solution | 2.87 |
| | 5°C/ambient RH | 1 month | Clear, colorless solution | 2.84 |
| | | 3 months | Clear, colorless solution | 2.87 |
| | | 6 months | Clear, colorless solution | 2.80 |
| | 25°C/60% RH | 1 month | Clear, colorless solution | 2.83 |
| | | 3 months | Clear, colorless solution | 2.87 |
| | | 6 months | Clear, color matches with 'Y5' ( Light Yellow) | 2.82 |
| | 40°C/75% RH | 1 month | Clear, colorless solution | 2.80 |
| | | 3 months | Clear, color matches with 'Y3' (Yellow) | 2.90 |
| | | 6 months | Clear, color matches with 'Y3' (Yellow) | 2.86 |

**Table 64. Chemical data: 5mg/mL, T110 1215, BO_300 BR**

| **Storage conditions** | | **Assay** | | | | **Degradation products** | | | | **Enantiomer** | |
|---|---|---|---|---|---|---|---|---|---|---|---|
| | | **Active ingredient** [%] | **Potassium sorbate** [%] | **Methyl** paraben [%] | **536-**11 [%] | 537-11 [%] | **0.42** RRT [%] | 0.43 **RRT** [%] | **0.48 RRT** [%] | **Total** [%] | **[%]** |
| **Requirements** | | **90.0** - **110.0** | **For information only** | **For information only** | **≤ 0.5** | **≤ 0.5** | **≤ 0.5** | **≤ 0.5** | **≤ 0.5** | **≤ 2.0** | **≤ 0.7** |
| | Initial analysis | 103.1 | 104.9 | 105.9 | <RT | <RT | <RT | <RT | <RT | <RT | 0.3 |
| 5°C/ambient RH | 1 month | 104.5 | 104.9 | 105.7 | <RT | <RT | <RT | <RT | <RT | <RT | 0.2 |
| | 3 months | 102.2 | 103.0 | 103.6 | <RT | <RT | <RT | <RT | <RT | <RT | 0.3 |
| | 6 months | 105.5 | 105.3 | 106.9 | <RT | <RT | <RT | <RT | <RT | <RT | 0.3² |
| 25°C/60% RH | 1 month | 103.9 | 102.5 | 105.1 | <RT | <RT | <RT | <RT | <RT | <RT | 0.2 |
| | 3 months | 104.1 ¹ | 101.5¹ | 104.1 ¹ | <RT | <RT | <RT | <RT | <RT | <RT | 0.3 |
| | 6 months | 105.3 | 102.1 | 105.9 | <RT | <RT | <RT | <RT | <RT | <RT | 0.3² |
| 40°C/75% RH | 1 month | 102.3 | 101.7 | 103.6 | <RT | <RT | <RT | <RT | <RT | <RT | 0.2 |
| | 3 months | 103.5 | 88.0 | 104.0 | <RT | <RT | <RT | <RT | <RT | <RT | 0.3 |
| | 6 months | 103.0 | 53.4 | 102.5 | <RT | <RT | <RT | <RT | <RT | <RT | 0.2² |

| | | **Active ingredient** [%] | **Potassium sorbate** [%] | **Methyl paraben** [%] | **536-11** [%] | **537-11** [%] | **0.42 RRT** [%] | **0.43 RRT** [%] | **0.48 RRT** [%] | **Total** [%] | [%] |
|---|---|---|---|---|---|---|---|---|---|---|---|
| **Requirements** | | **90.0** - **110.0** | **For information only** | **For information only** | ≤ **0.5** | ≤ **0.5** | ≤ **0.5** | ≤ **0.5** | ≤ **0.5** | ≤ **2.0** | ≤ **0.7** |
| | Initial analysis | 103.1 | 104.9 | 105.9 | <RT | <RT | <RT | <RT | <RT | <RT | 0.3 |

| | | | | | | | | | | | |
|---|---|---|---|---|---|---|---|---|---|---|---|
| ¹ Reanalysis results are reports, for details refer AQWA # 1567 835. ² For enantiomer test flow rate is not followed as per test method (1.0ml/minute is followed instead of 0.8ml/min ), for details refer to deviation # 1618342. | | | | | | | | | | | |

**Table 65. Physical data: 5mg/mL, T110 1215, BO_300BR**

| **Storage conditions** | | | **Appearance** | **pH value** |
|---|---|---|---|---|
| **Requirements** | | | **For information only** | **For information only** |
| | | Initial analysis | Clear, colorless solution | 2.86 |
| | 5°C/ambient RH | 1 month | Clear, colorless solution | 2.82 |
| | | 3 months | Clear, colorless solution | 2.86 |
| | | 6 months | Clear, colorless solution | 2.84 |
| | 25°C/60% RH | 1 month | Clear, colorless solution | 2.83 |
| | | 3 months | Clear, colorless solution | 2.86 |
| | | 6 months | Clear, colorless solution | 2.85 |
| | 40°C/75% RH | 1 month | Clear, color matches with 'Y6' (light yellow) | 2.84 |
| | | 3 months | Clear, Clear, color matches with 'Y4' (Yellow) | 2.87 |
| | | 6 months | Clear, Clear, color matches with 'Y3' (Yellow) | 2.85 |

**Table 66. Chemical data: 5mg/mL, T112 1215, BO_300BR**

| **Storage conditions** | | **Assay** | | | **Degradation products** | | | | | | **Enantiomer** |
|---|---|---|---|---|---|---|---|---|---|---|---|
| | | **Active ingredient** [%] | **Potassium sorbate** [%] | **Methyl paraben** [%] | **536-11** [%] | **537-11** [%] | **0.42 RRT** [%] | **0.43 RRT** [%] | **0.48 RRT** [%] | **Total** [%] | [%] |
| **Requirements** | | **90.0 - 110.0** | For information only | For information only | **≤ 0.5** | ≤**≤ 0.5** | **≤ 0.5** | **≤ 0.5** | **≤ 0.5** | **≤ 2.0** | **≤ 0.7** |
| | Initial analysis | 100.7 | 101.8 | 102.2 | <RT | <RT | <RT | <RT | <RT | <RT | 0.3 |
| 5°C/ambient RH | 1 month | 101.1 | 101.7 | 102.2 | <RT | <RT | <RT | <RT | <RT | <RT | 0.1 |
| | 3 months | 99.9 | 99.9 | 100.2 | <RT | <RT | <RT | <RT | <RT | <RT | 0.3 |
| | 6 months | 102.8 | 102.8 | 104.1 | <RT | <RT | <RT | <RT | <RT | <RT | 0.3² |
| 25°C/60% RH | 1 month | 102.4 | 91.4 | 105.6 | <RT | <RT | <RT | <RT | <RT | <RT | 0.2 |
| | 3 months | 103.4 | 86.4 | 106.4 | <RT | <RT | <RT | <RT | <RT | <RT | 0.2 |
| | 6 months | 103.7 | 60.9 | 107.6 | <RT | <RT | <RT | <RT | <RT | <RT | 0.2² |
| 40°C/75% RH | 1 month | 101.4 | 96.0 | 102.0 | <RT | <RT | <RT | <RT | <RT | <RT | 0.1 |
| | 3 months | 102.1 ¹ | 69.3 ¹ | 104.5 ¹ | <RT | <RT | <RT | <RT | <RT | <RT | 0.2 |
| | 6 months | 100.6 | 18.8 | 104.7 | <RT | <RT | <RT | <RT | <RT | <RT | 0.2² |
| **Requirements** | | **90.0** - **110.0** | **For information only** | **For information only** | ≤ **0.5** | ≤ **0.5** | ≤ **0.5** | ≤ **0.5** | ≤ **0.5** | ≤ **2.0** | ≤ **0.7** |
| | Initial analysis | 100.7 | 101.8 | 102.2 | <RT | <RT | <RT | <RT | <RT | <RT | 0.3 |

| | | | | | | | | | | | |
|---|---|---|---|---|---|---|---|---|---|---|---|
| ¹ Reanalysis results are reports, for details refer AQWA # 1572599. ² For enantiomer test flow rate is not followed as per test method (1.0ml/minute is followed instead of 0.8ml/min ), for details refer to deviation # 1618342 | | | | | | | | | | | |

**Table 67. Physical data: 5mg/mL, T112 1215, BO_300BR**

| **Storage conditions** | | | **Appearance** | **pH value** |
|---|---|---|---|---|
| **Requirements** | | | **For information only** | **For information only** |
| | | Initial analysis | Clear, colorless solution | 2.82 |
| | 5°C/ambient RH | 1 month | Clear, colorless solution | 2.83 |
| | | 3 months | Clear, colorless solution | 2.87 |
| | | 6 months | Clear, colorless solution | 2.83 |
| | 25°C/60% RH | 1 month | Clear, colorless solution | 2.81 |
| | | 3 months | Clear, color matches with 'Y5' (light yellow) | 2.85 |
| | | 6 months | Clear, color matches with 'Y4' (Yellow) | 2.80 |
| | 40°C/75% RH | 1 month | Clear, color matches with 'Y6' (light yellow) | 2.83 |
| | | 3 months | Clear, color matches with 'Y2' (light yellow) | 2.85 |
| | | 6 months | Clear, color matches with 'Y1' ( Dark yellow) | 2.81 |

### Microbiological data

**Table 68. Microbiological data for RUXOLITINIB 1mg/mL, 5mg/mL solution**

| **Batch, Strength, Packaging** | **Storage condition** | **Total aerobic microbial count (TAMC) [cfu/ml]** | **Total yeasts and molds count (TYMC) [cfu/ml]** | **Specific microorganisms: Escherichia coli** |
|---|---|---|---|---|
| **Requirement** | | **<=200 cfu/ml** | **<=20 cfu/ml** | **Absent in 1ml** |
| T109 1215 1mg/mL, BO_125_AMBER_CR_CLOSURE | Initial analysis | <10 cfu/ml | <10 cfu/ml | Absent in 1ml |
| T109 1215 1mg/mL, BO_125_AMBER_CR_CLOSURE | 6 months (25°C/60% RH) | <10 cfu/ml | <10 cfu/ml | Absent in 1ml |
| T111 1215 1mg/mL, BO_125_AMBER_CR_CLOSURE | Initial analysis | <10 cfu/ml | <10 cfu/ml | Absent in 1ml |
| T111 1215 1mg/mL, BO_125_AMBER_CR_CLOSURE | 6 months (25°C/60% RH) | <10 cfu/ml | <10 cfu/ml | Absent in 1ml |
| T110 1215 5mg/mL, BO_125_AMBER_CR_CLOSURE | Initial analysis | <10 cfu/ml | >100 cfu/ml¹ | Absent in 1ml |
| T110 1215 5mg/mL, BO_125_AMBER_CR_CLOSURE | 6 months (5°C) | <10 cfu/ml | <10 cfu/ml | Absent in 1ml |
| T110 1215 5mg/mL, BO_125_AMBER_CR_CLOSURE | 6 months (25°C/60% RH) | <10 cfu/ml | <10 cfu/ml | Absent in 1ml |
| T112 1215 5mg/mL, BO_125_AMBER_CR_CLOSURE | Initial analysis | <10 cfu/ml | <10 cfu/ml | Absent in 1ml |
| T112 1215 5mg/mL, BO_125_AMBER_CR_CLOSURE | 6 months (25°C/60% RH) | <10 cfu/ml | <10 cfu/ml | Absent in 1ml |
| T109 1215 1mg/mL, BO_300BR | - | - | - | - |
| T111 1215 1mg/mL, BO_300BR | - | - | - | - |
| T110 1215 5mg/mL, BO_300BR | - | - | - | - |
| T112 1215 5mg/mL, BO_300BR | - | - | - | - |
| - Not performed | | | | |
| ¹ MET failed, for details refer deviation # 1606983 | | | | |

## Claims

1. An oral formulation comprising:
(i) ruxolitinib or a pharmaceutically acceptable salt thereof,
(ii) water,
(iii) a pH adjusting agent, and
(iv) a preservative,
wherein the ruxolitinib concentration in the oral formulation is from about 0.1mg/mL to about 10 mg/mL on a free base basis, and wherein the pH of the oral formulation is 2.7±0.5.

2. The oral formulation of claim 1, wherein the preservative is mixture of methyl paraben and propyl paraben.

3. The oral formulation of claim 1 or claim 2, further comprising a co-solvent.

4. The oral formulation of claim 3, wherein the co-solvent is propylene glycol.

5. The oral formulation of any one of the preceding claims, further comprising a sweetener.

6. The oral formulation of claim 5, wherein the sweetener is sucralose.

7. The oral formulation of any one of the preceding claims, further comprising a flavouring agent.

8. The oral formulation of claim 7, wherein flavouring agent is strawberry flavour.

9. The oral formulation of any one of the preceding claims, wherein the pH-adjusting agent is citric acid.

10. The oral formulation of any one of the preceding claims, wherein the ruxolitinib (on a free base basis) concentration is about 1 mg/mL to 5mg/mL.

11. An oral formulation at 5mg/mL ruxolitinib concentration comprising
| **Component** | **Functionality** | **Amount per unit (mg/mL)** |
|---|---|---|
| Ruxolitinib phosphate* | Active ingredient | 6.600 |
| Propylene glycol | Co-solvent | 150.000 |
| Methyl paraben | Preservative | 1.200 |
| Propyl paraben | Preservative | 0.400 |
| Sucralose | Sweetener | 2.000 |
| Citric acid | pH-adjusting agent | 4.270 |
| Strawberry flavor | Flavor | 2.500 |
| Purified water | Vehicle | 848.030 |
| Total | | q.s to 1mL |
| | | |
|---|---|---|
| * Salt factor: 1.320 | | |

12. An oral formulation at 1mg/mL ruxolitinib concentration comprising
| **Component** | **Functionality** | **Amount per unit (mg/mL)** |
|---|---|---|
| Ruxolitinib phosphate* | Active ingredient | 1.320 |
| Propylene glycol | Co-solvent | 150.000 |
| Methyl paraben | Preservative | 1.200 |
| Propyl paraben | Preservative | 0.400 |
| Sucralose | Sweetener | 2.000 |
| Citric acid | pH-adjusting agent | 4.270 |
| Strawberry flavor | Flavor | 2.500 |
| Purified water | Vehicle | 853.310 |
| Total | | q.s to 1mL |
| | | |
|---|---|---|
| * salt factor 1.320 | | |

13. The formulation according to any one of the preceding claims, for use in a method of treating a Janus kinase (JAK) associated disease, the method comprising the step of administering the oral dosage form according to any one of the preceding claims , comprising about 5 mg to about 65 mg of ruxolitinib, on a free base basis, or pharmaceutically acceptable salt thereof.

14. The formulation for use according to claim 13, wherein the JAK associated disease is myelofibrosis (MF), polycythemia vera (PV) or graft-versus-host disease (GvHD).

## Patentansprüche

1. Orale Formulierung, umfassend:
(i) Ruxolitinib oder ein pharmazeutisch unbedenkliches Salz davon,
(ii) Wasser,
(iii) ein Mittel zur Einstellung des pH-Werts und
(iv) ein Konservierungsmittel,
wobei die Ruxolitinib-Konzentration in der oralen Formulierung etwa 0,1 mg/ml bis etwa 10 mg/ml bezogen auf die freie Base beträgt und wobei der pH-Wert der oralen Formulierung 2,7±0,5 beträgt.

2. Orale Formulierung nach Anspruch **1,** wobei es sich bei dem Konservierungsmittel um eine Mischung aus Methylparaben und Propylparaben handelt.

3. Orale Formulierung nach Anspruch 1 oder Anspruch 2, ferner umfassend ein Cosolvens.

4. Orale Formulierung nach Anspruch 3, wobei es sich bei dem Cosolvens um Propylenglykol handelt.

5. Orale Formulierung nach einem der vorhergehenden Ansprüche, ferner umfassend ein Süßungsmittel.

6. Orale Formulierung nach Anspruch 5, wobei es sich bei dem Süßungsmittel um Sucralose handelt.

7. Orale Formulierung nach einem der vorhergehenden Ansprüche, ferner umfassend einen Geschmacksstoff.

8. Orale Formulierung nach Anspruch 7, wobei der Geschmacksstoff Erdbeergeschmack ist.

9. Orale Formulierung nach einem der vorhergehenden Ansprüche, wobei es sich bei dem Mittel zur Einstellung des pH-Werts um Citronensäure handelt.

10. Orale Formulierung nach einem der vorhergehenden Ansprüche, wobei die Ruxolitinib-Konzentration (bezogen auf die freie Base) etwa 1 mg/ml bis 5 mg/ml beträgt.

11. Orale Formulierung mit einer Ruxolitinib-Konzentration von 5 mg/ml, umfassend
| Komponente | Funktionalität | Menge pro Einheit (mg/ml) |
|---|---|---|
| Ruxolitinibphosph at* | Wirkstoff | 6,600 |
| Propylenglykol | Co-Lösungsmittel | 150,000 |
| Methylparaben | Konservierungsstoff | 1,200 |
| Propylparaben | Konservierungsstoff | 0,400 |
| Sucralose | Süßungsmittel Mittel zur | 2,000 |
| Citronensäure | Einstellung des pH Werts | - 4,270 |
| Erdbeergeschmack | Geschmack | 2,500 |
| Gereinigtes Wasser Vehikel | | 848,030 |
| Insgesamt | | q.s. bis 1 ml |
| | | |
|---|---|---|
| * Salzfaktor: 1,320 | | |

12. Orale Formulierung mit einer Ruxolitinib-Konzentration von 1 mg/ml, umfassend
| Komponente | Funktionalität | Menge pro Einheit (mg/ml) | |
|---|---|---|---|
| Ruxolitinibphosph at* | Wirkstoff | 1,320 | |
| Propylenglykol | Co-Lösungsmittel | 150,000 | |
| Methylparaben | Konservierungsstoff | 1,200 | |
| Propylparaben | Konservierungsstoff | 0,400 | |
| Sucralose | Süßungsmittel | 2,000 | |
| | Mittel | zur | |
| Citronensäure | Einstellung des Werts | pH-4,270 | |
| Erdbeergeschmack | Geschmack | | 2,500 |
| Gereinigtes Wasser Vehikel | | | 853,310 |
| Insgesamt | | | q.s. bis 1 ml |
| | | | |
|---|---|---|---|
| * Salzfaktor: 1,320 | | | |

13. Formulierung nach einem der vorhergehenden Ansprüche zur Verwendung bei einem Verfahren zur Behandlung einer mit Janus-Kinase (JAK) assoziierten Erkrankung, wobei das Verfahren den Schritt des Verabreichens der oralen Dosierungsform nach einem der vorhergehenden Ansprüche umfasst, die etwa 5 mg bis etwa 65 mg Ruxolitinib bezogen auf die freie Base oder ein pharmazeutisch unbedenkliches Salz davon umfasst.

14. Formulierung zur Verwendung nach Anspruch 13, wobei es sich bei der JAK-assoziierten Erkrankung um Myelofibrose (MF), Polycythemia Vera (PV) oder Graft-versus-Host-Krankheit (GvHD) handelt.

## Revendications

1. Formulation orale comprenant :
(i) du ruxolitinib ou un sel pharmaceutiquement acceptable de celui-ci ;
(ii) de l'eau,
(iii) un agent d'ajustement du pH, et
(iv) un conservateur,
la concentration de ruxolitinib dans la formulation orale étant d'environ 0,1 mg/mL à environ 10 mg/mL sur une base de base libre, et le pH de la formulation orale étant de 2,7 ± 0,5.

2. Formulation orale selon la revendication 1, dans laquelle le conservateur est un mélange de méthylparabène et de propylparabène.

3. Formulation orale selon la revendication 1 ou la revendication 2, comprenant en outre un co-solvant.

4. Formulation orale selon la revendication 3, dans laquelle le co-solvant est le propylène glycol.

5. Formulation orale selon l'une quelconque des revendications précédentes, comprenant en outre un édulcorant.

6. Formulation orale selon la revendication 5, dans laquelle l'édulcorant est le sucralose.

7. Formulation orale selon l'une quelconque des revendications précédentes, comprenant en outre un agent aromatisant.

8. Formulation orale selon la revendication 7, dans laquelle l'agent aromatisant est un arôme de fraise.

9. Formulation orale selon l'une quelconque des revendications précédentes, dans laquelle l'agent d'ajustement du pH est l'acide citrique.

10. Formulation orale selon l'une quelconque des revendications précédentes, dans laquelle la concentration de ruxolitinib (sur une base de base libre) est d'environ 1 mg/mL à 5 mg/mL.

11. Formulation orale à une concentration de ruxolitinib de 5 mg/mL comprenant
| Composant | Fonctionnalité | | Quantité par unité (mg/mL) |
|---|---|---|---|
| Phosphate ruxolitinib* | deIngrédient actif | | 6,600 |
| Propylèneglycol | | Co-solvant | 150,000 |
| Méthylparabène | | Conservateur | 1,200 |
| Propylparabène | | Conservateur | 0,400 |
| Sucralose | | Édulcorant | 2,000 |
| Acide citrique | | Agent d'ajustement du pH | 4,270 |
| Arôme de fraise | | Arôme | 2,500 |
| Eau purifiée | | Véhicule | 848,030 |
| Total | | | qsp à 1 mL |
| | | | |
|---|---|---|---|
| * Facteur de sel : 1,320 | | | |

12. Formulation orale à une concentration de ruxolitinib de 1 mg/mL comprenant
| Composant | Fonctionnalité | | Quantité par unité (mg/mL) |
|---|---|---|---|
| Phosphate ruxolitinib* | deIngrédient actif | | 1,320 |
| Propylèneglycol | | Co-solvant | 150,000 |
| Méthylparabène | | Conservateur | 1,200 |
| Propylparabène | | Conservateur | 0,400 |
| Sucralose | | Édulcorant | 2,000 |
| Acide citrique | Agent d'ajustement du pH | 4,270 | |
| Arôme de fraise | Arôme | 2,500 | |
| Eau purifiée | Véhicule | 853,310 | |
| Total | | qsp à 1 mL | |
| | | | |
|---|---|---|---|
| * Facteur de sel : 1,320 | | | |

13. Formulation selon l'une quelconque des revendications précédentes, pour utilisation dans un procédé de traitement d'une maladie associée à la Janus kinase (JAK), le procédé comprenant l'étape d'administration de la forme posologique orale selon l'une quelconque des revendications précédentes, comprenant environ 5 mg à environ 65 mg de ruxolitinib, sur une base de base libre, ou d'un sel pharmaceutiquement acceptable de celui-ci.

14. Formulation pour utilisation selon la revendication 13, dans laquelle la maladie associée à la JAK est la myélofibrose (MF), la polycythémie vraie (PV) ou la maladie du greffon contre l'hôte (GvHD).
